# EUROPEAN PATENT SPECIFICATION

(11) **EP 1 366 052 B1**
(45) Date of publication and mention of the grant of the patent: **22.03.2006**
(21) Application number: 02715338.6
(22) Date of filing: 11.01.2002
(51) Int. Cl.: C07D 493/10

(54) **NOVEL MULTI-RING ORGANIC COMPOUNDS FOR REGULATING GUT MOTILITY AND FOOD INTAKE**
NEUE POLYCYLISCHE ORGANISCHE VERBINDUNGEN ZUR REGULIERUNG DER DARMTÄTIGKEIT UND DER NAHRUNGSAUFNAHME
NOUVEAUX COMPOSES ORGANIQUES A NOYAUX MULTIPLES POUR REGULER LE TRANSIT INTESTINAL ET EQUILIBRER LA RATION ALIMENTAIRE

(30) Priority: 11.01.2001 US 261019 P
(43) Date of publication of application: 03.12.2003
(73) Proprietor: Enpharma L.P., Hamilton, HM 11 (BM)
(72) Inventor: KRANTIS, Anthony, Ottawa, Ontario K1Y 0Z3 (CA); DURST, Tony, Ottawa, Ontario K1H 6W2 (CA)
(74) Representative: Brady, Paul Andrew
(86) International application number: PCT/CA2002/000025
(87) International publication number: WO 2002/055522

(56) References cited:
- WO-A-01/01968
- US-A- 5 859 051

## Description

### GENERAL FIELD OF THE INVENTION

This invention is generally in the field of treating obesity and regulating food intake. In particular, this invention relates to compositions and uses of the compositions for regulating gut motility and treating obesity using novel multi-ring organic compounds.

### BACKGROUND OF THE INVENTION

Overeating leading to obesity is a major health problem. Obesity increases the risk of diabetes, heart disease, cancer and other chronic diseases, in addition to increasing the physical or mechanical restrictions imposed on the body. Although such adverse health effects of obesity are scientifically well documented and generally well understood by the public, the effective control of appetite and overeating on an individual basis has been a goal-difficult to attain for millions of people. Approximately 25 percent of the children in North America are considered overweight or obese. North Americans alone spend approximately $40 billion per annum on weight loss treatments, and this amount appears to be increasing. A recent study conservatively estimated the annual cost of treating obesity in Canada at $1.8 billion, representing 2.4 percent of the total health care expenditures for all diseases (see, "Cost of Obesity: $1.8 billion," in *Pharmaceutical Manufacturers Association of Canada,* March 1999, page 11).

Currently available anti-obesity drugs work for the most part by targeting central nervous system (CNS) pathways to induce appetite suppression. However, such drugs have a number of CNS-related side effects, such as anxiety, and there is the potential for chronic health problems such as hypertension, cardiovascular disease, and diabetes. Another current approach to treating obesity is to control appetite by using "bulk" products, which are ingested instead of normal food. Such bulk products have the problem of altering nutritional status in that the bulk product does not contain the necessary range of desirable nutrients. Moreover, the individual who ingests a bulk product may refuse to consume any food, even desirable nutrients.

Drugs that suppress appetite are among the least desirable means to treat obesity because weight is usually regained once administration of such drugs is halted. Furthermore, serious undesirable side effects, including increased risk of diseases such as primary pulmonary hypertension may limit the use of such drugs. For example, the appetite suppressants fenfluramine and dexfenfluramine were recently pulled off the market by their manufacturers because of a potential for serious adverse effects on the lungs and heart.

Another type of obesity treatment that has emerged recently is the use of drugs that interfere with fat absorption from the small intestine. Such a drug may, for example, inhibit pancreatic enzymes used for fat digestion. Undigested fat is then passed through the intestines and excreted. Decreasing fat absorption can result in oily stool, oily spotting of undergarments, intestinal gas, frequent bowel movements, and decrease absorption of fat-soluble nutrients such as vitamins A, D, and E.

There is currently no medical approach that cuts weight gain without unhealthy side effects or increased risk of disease. Needs remain for effective compositions and methods for controlling food intake and treating excessive weight gain, as in obesity, in humans and other animals without untoward nutritional and medical side effects.

### SUMMARY OF THE INVENTION

This invention provides compositions useful for controlling food intake in humans and other animals. In particular, the invention provides non-naturally occurring compositions comprising heretofore unknown, multi-ring, organic compounds structurally related to a group of organic compounds known as trichothecenes that are capable of regulating gut motility in humans and other animals. Some compounds described herein induce a pattern of gut motor activity (fed pattern) that signals satiety in humans and other animals and may be used to reduce food intake by an individual. Other compounds may intensify or enhance a pattern of gut motor activity (fasting pattern) that characterizes the unfed or fasting state of a human or other animal and may be used to stimulate food uptake by an individual or to counteract or modulate the effect of other compounds that induce the fed pattern.

Recent discoveries have begun to elucidate how certain naturally occurring trichothecene mycotoxins (found in crops, feed, and food contaminated with certain fungal species) are capable of inducing satiety and feed refusal in humans and other vertebrate animals, respectively, and also the neural circuitry regulating patterns of gut motor activity ("gut motility"), which propels food through the organs of the gut (see, United States Provisional Application No. 60/143,054, filed July 6, 1999 and international PCT Application No. PCr/CA00/00790, filed July 6 2000 published as WO 01/01968, which describes methods and compositions for regulating gut motility and food intake; incorporated herein by reference). Uses of compounds of the invention involve administering a compound described herein that affects the pattern of gut motility, that is, the pattern of contractions, relaxations, and quiescence of the smooth muscle tissue of the organs of the gut and, thereby, affect food intake. Stimulating the "fed pattern" of gut motor activity signals satiety, that is, a feeling of fullness, which shortens the time an individual spends eating or feeding. Thus, compounds that stimulate the fed pattern of gut motility are useful for treatment where the goal is to limit food intake, as in treating obesity. In contrast, compounds that enhance or intensify the "fasting pattern" or interfere with the fed pattern of gut motility will tend to increase eating or feeding time because satiety is not signaled to the body.

In one embodiment, the pharmaceutical compositions of the invention comprise a gut motility regulating "8-O-substituted-7α,15-carbonate compound" of formula I, as shown below (including selected trichothecene-type numbering for various atoms and groups in the chemical structure): 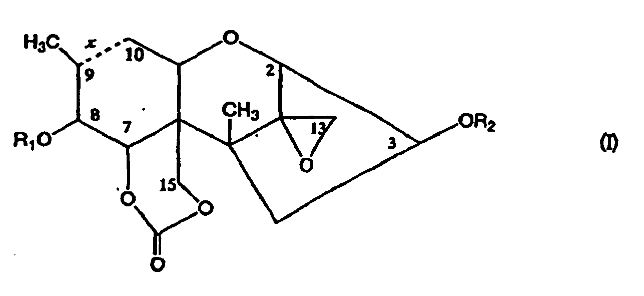 wherein, x is a single or double bond between carbon atom-9 and carbon atom-10; R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; or
   a 5- or 6-membered heteroaromatic ring substituted with a substituent selected from the group consisting of halogen, alkyl (e.g., methyl), alkoxy (e.g, methoxy), thiomethyl, sulfinylmethyl, sulfonylmethyl, and combinations thereof.
   Preferred representative species of formula I include 3α-acetoxy-12,13-epoxy-8α-hydroxytrichothec-9-en-7α,15-carbonate (designated EN139499); 3α,8α-diacetoxy-12,13-epoxytrichothec-9-en-7α,15-carbonate (designated EN139500); and 3α,8α-diacetoxy-12,13-epoxy-9α-methyltrichothecan-7α,15-carbonate (designated EN139507).

In another embodiment, the pharmaceutical compositions of the invention comprise a gut motility regulating "acetal compound" of formula II, as shown below (including selected trichothecene-type numbering for various atoms and groups in the chemical structure): 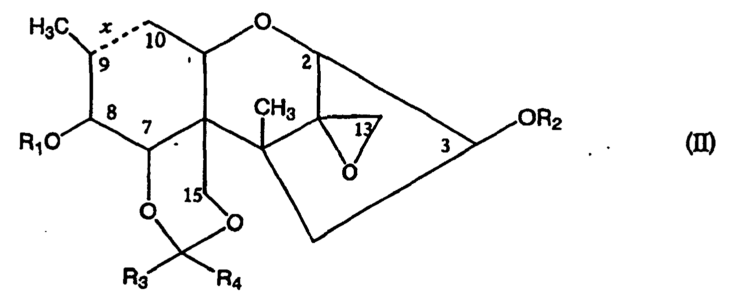 wherein x is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; or
   a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof;
R₃ and R₄ are, independently:
a hydrogen;
a C₁ to C₆ alkyl;
a phenyl group;
a phenyl group substituted with a substituent selected from the group consisting
of halogen, alkyl (for example, methyl), alkoxy (for example, methoxy), thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Preferred representative species of formula II include 3α-acetoxy-7α, 15-benzylidene-12,13-epoxy-9α-methyltrichothecan-8α-ol (designated EN139501) and 7α,15-benzylidene-3α,8α-diacetoxy-12,13-epoxy-9α-methyltrichothecane (designated EN139505).

In another embodiment, the pharmaceutical compositions of the invention comprise a gut motility regulating "7α,15-diol" compound of formula III, as shown below (including selected trichothecene-type numbering for various atoms and groups in the chemical structure): 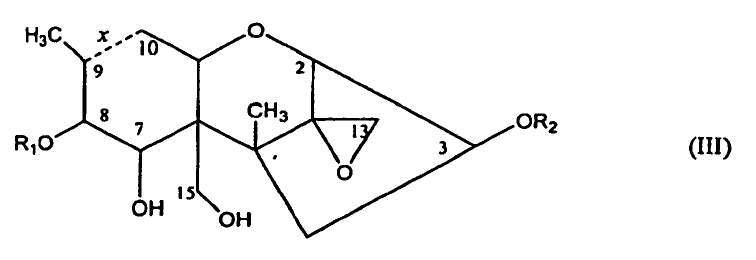 wherein x is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring;
   a 5- or 6-membered heteroaromatic ring substituted with a substituent selected from the group consisting of halogen, alkyl (for example, methyl), alkoxy (for example, methoxy), thiomethyl, sulfinylmethyl, suffonylmethyl, and combinations thereof.
Preferred representative species of formula III include 3α-acetoxy-12,13-epoxy-9α-methyltrichothecan-7α,8α,15-triol (designated EN 139503) and 3α,8α-diacetoxy-12,13-epoxy-9α-methyltrichothecan-7α,15-diol (designated EN139506).

In another embodiment, the pharmaceutical compositions of the invention comprise a gut motility regulating 8-keto-7α,15-carbonate compound of formula IV, as shown below (including selected trichothecene-type numbering for various atoms and groups in the chemical structure) 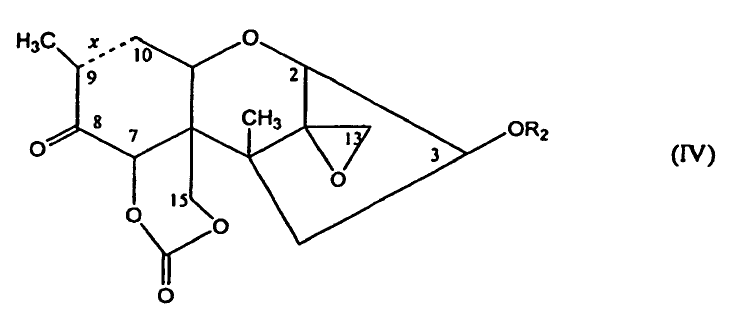 wherein x is a single or a double bond between carbon atom-9 and carbon atom-10;
R₂ is:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and
   a 5- or 6-membered heteroaromatic ring substituted with a substituent selected from the group consisting of halogen, alkyl (for example, methyl), alkoxy (for example, methoxy), thiomethyl, sulfinymethyl, sulfonylmethyl, and combinations thereof; with the provise that when x is a double bond, then R₂ is not hydrogen or C(O)R₅ wherein R₅ is methyl.
Preferred representative species of formula IV include 3α-acetoxy-12, 13-epoxy-9α-methyltrichothecan-8-one-7α,15-carbonate (designated EN139511) and 3α-benzoyloxy-12,13-epoxytrichothec-9-en-8-on-7α,15 carbonate (designated EN139514).

In another embodiment, the pharmaceutical compositions of the invention comprise a gut motility regulating 8-keto-12,13-epoxy-compound of formula V, as shown below: 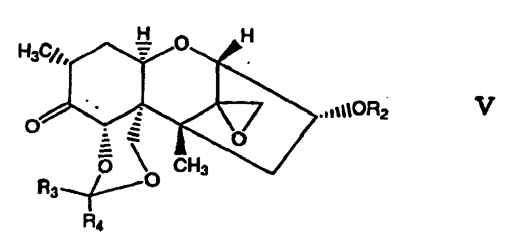 wherein R₂ is:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and
   a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof; and
R₃ and R₄ are, independently:
a hydrogen;
a C₁ to C₆ alkyl;
a phenyl group;
a phenyl group substituted with a substituent selected from the group consisting of halogen, alkyl (for example, methyl), alkoxy (for example, methoxy), thilomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Preferred representative species of formula V include, without limitation, 3-α-acetoxy-7α,15-benzylidene-12,13-epoxytrichothecan-8-one (designated EN139519) and 7α,15-benzylidene-12,13-epoxy-3β-hydroxytrichothecan-8-one (designated EN139520).

In another embodiment, the pharmaceutical compositions of the invention comprise a gut motility regulating 8-keto-12α-hydroxy compound of formula VI as shown below: 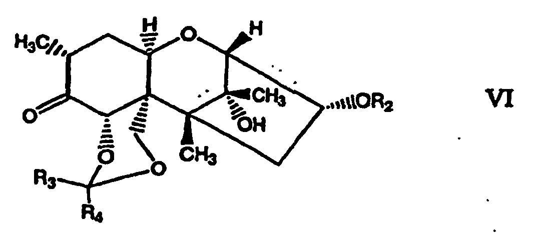 wherein R₂ is:
a hydrogen atom;
a C₁ to C₆ alkyl:
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof; and
R₁ and R₄ are, independently:
a hydrogen;
a C₁ to C₆ alkyl;
a phenyl group;
a phenyl group substituted with a substituent selected from the group consisting of halogen, alkyl (for example, methyl), alkoxy (for example, methoxy), thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Preferred representative species of formula VI include, without limitation, 7α,15-benzylidene-9α,12β-dimethyl-3α,12α-dihydroxytrichothecan -8-one (designated EN139522).

Another compound that is useful in the pharmaceutical compositions and uses of the invention is 12,13-epoxytrichothec-9-ene-3α,7α,8α,15-tetraol (designated EN139518).

Another aspect of the invention provides pharmaceutical compositions comprising any of the gut motility regulating compounds described herein and a pharmaceutically acceptable carrier.

The invention also provides compounds useful for regulating food intake and treating obesity in an individual comprising administering to the individual a composition described herein in a dose sufficient to induce satiety or feed refusal in the individual. In such uses a composition of the invention may be administered in any of a variety of routes. Preferably, a composition of the invention is administered to an individual orally.

### BRIEF DESCRIPTION OF THE DRAWINGS

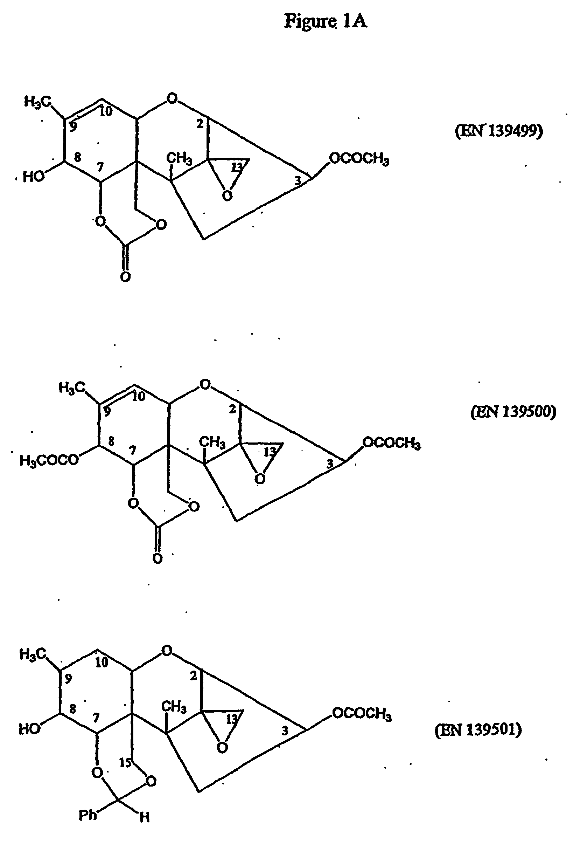

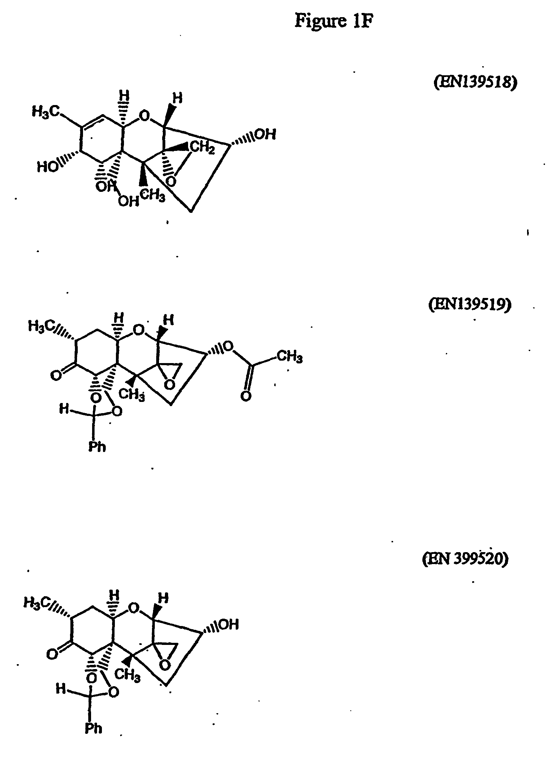

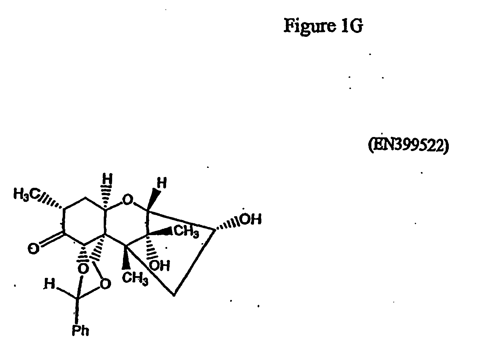

Figures 1A-1G show molecular structures and designations of some representative compounds of the invention: EN139499, EN139500, EN139501, EN139502, EN139503, EN139504, EN139505, EN139506, EN139507, EN139508, EN139511, EN139514, EN139518, EN139519, EN139520, and EN139522. For clarity, only selected hydrogen atoms (H) present in the molecules are depicted on the structures in the figures. Ph = phenyl.

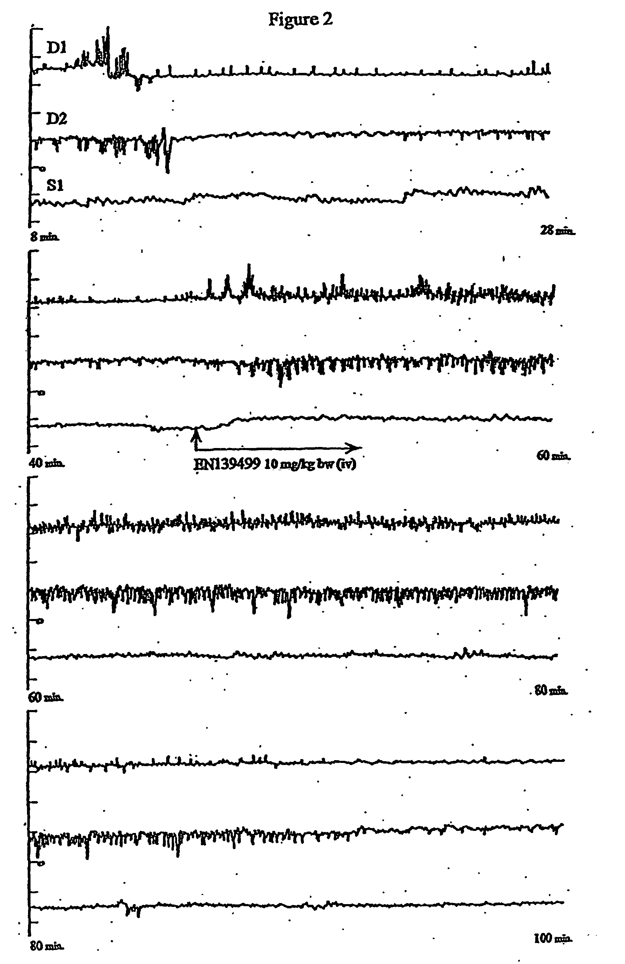

Figure 2 shows a recording of induction of the fed pattern of gut motor activity at two sites in the duodenum (D1 and D2) and at a site in the gastric antrum (S1) of an anaesthetized Sprague-Dawley male rat treated with EN139499 (10 mg·kg⁻¹ body weight, intravenous administration) by the method of Krantis et al. (1996). Figure 2 also shows the characteristic fasting pattern of gut motor activity with its alternating grouped and intergroup activities in the duodenum (D1 and D2) in the early portion (between t = 8 and t = 40 minutes) of the recording prior to administration of compound EN139499. Administration of EN139499 (vertical arrow) was followed (horizontal arrow) by a characteristic fed pattern of gut motor activity as shown in the duodenum recordings (D1, D2) as an induction of an intense pattern of hyperactivity and simultaneously in the gastric antrum (S1) as a measurable suppression or decrease in recorded tissue motor activity.

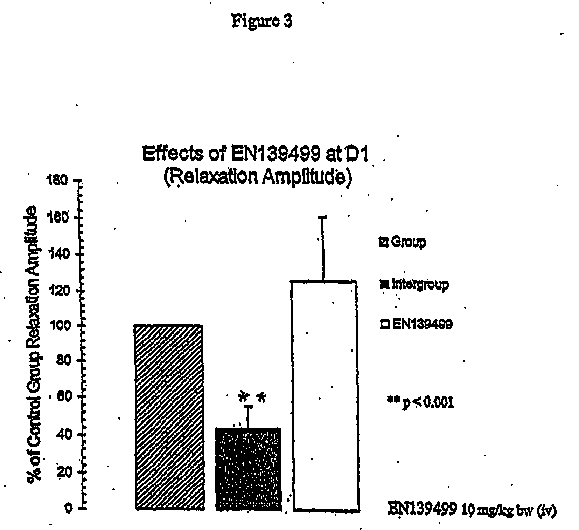

Figure 3 shows bar graphs of the effect on the amplitude of the relaxation component of gut motor activity recorded at duodenal site D1 of anaesthetized rats treated with compound EN139499 (10 mg·kg⁻¹ body weight, intravenous administration). Amplitude of relaxation is expressed as percent of control "group" activity. Bars show the relative amplitudes of the relaxation component of motor activity for "group" motor activity prior to administration of EN139499 (diagonal bar, 100%); for "intergroup" motor activity prior to administration of EN139499 (filled bar); and for stimulation of activity after administration of EN139499 (open bar).

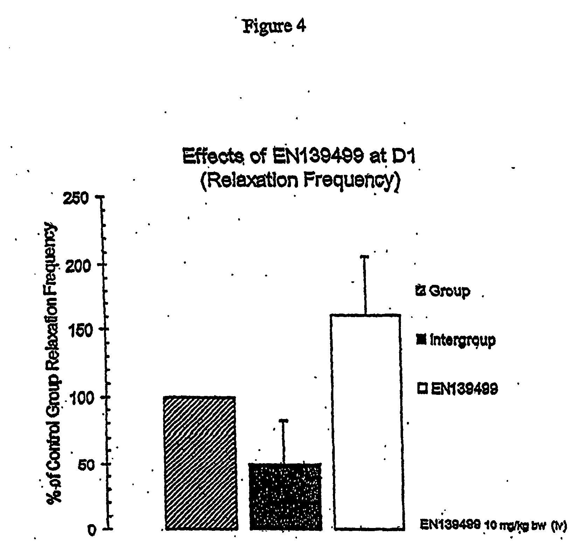

Figure 4 shows bar graphs of the effect on the frequency of the relaxation component of gut motor activity at duodenal site D1 of anaesthetized rats treated with compound EN139499 (10 mg·kg⁻¹ body weight, intravenous administration). Frequency of relaxation is expressed as percent of control "group" activity. Bars show the relative frequency of the relaxation component of motor activity for "group" motor activity prior to administration of EN139499 (diagonal bar, 100%); for "intergroup" motor activity prior to administration of EN139499 (filled bar); and for stimulation of activity after administration of EN139499 (open bar).

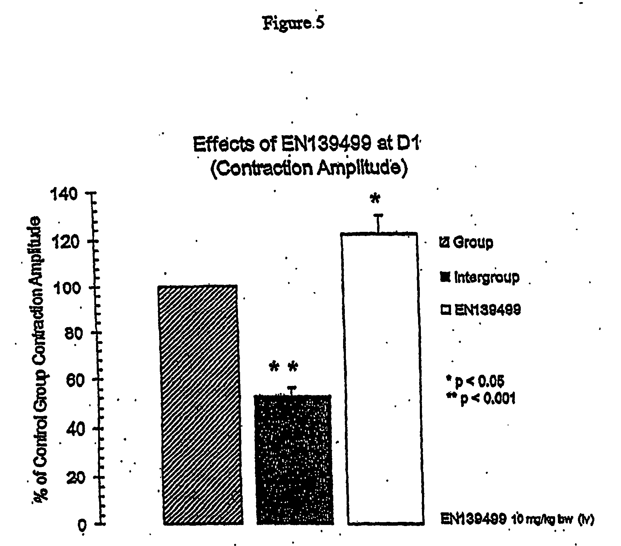

Figure 5 shows bar graphs of the effect on the amplitude of the contraction component of gut motor activity at duodenal site D1 of anaesthetized rats treated with compound EN139499 (10 mg-kg⁻¹ body weight, intravenous administration). Amplitude of contraction is expressed as percent of control "group" activity. Bars show the relative amplitude of the contraction component of motor activity for "group" motor activity prior to administration of EN139499 (diagonal bar, 100%); for "intergroup" motor activity prior to administration of EN139499 (filled bar); and for stimulation of activity after administration of EN139499 (open bar).

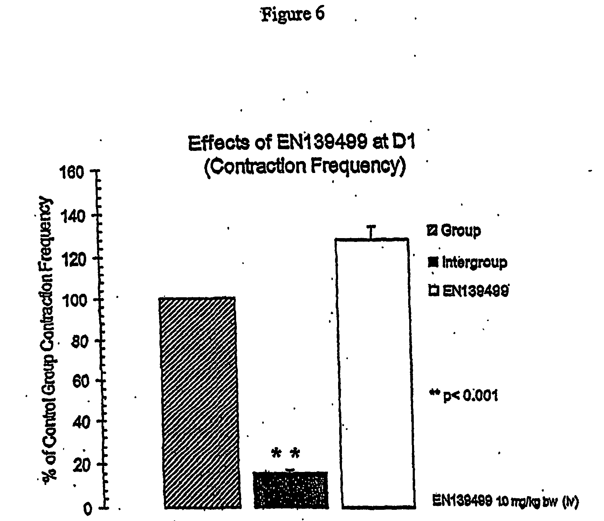

Figure 6 shows bar graphs of the effect on the frequency of the contraction component of gut motor activity at duodenal site D1 of anaesthetized rats treated with compound EN139499 (10 mg·kg-¹ body weight, intravenous administration). Frequency of contraction is expressed as percent of control "group" activity. Bars show the relative frequency of the contraction component of motor activity for "group" motor activity prior to administration of EN139499 (diagonal bar, 100%); for "intergroup" motor activity prior to administration of EN139499 (filled bar); and for stimulation of activity after administration of EN139499 (open bar).

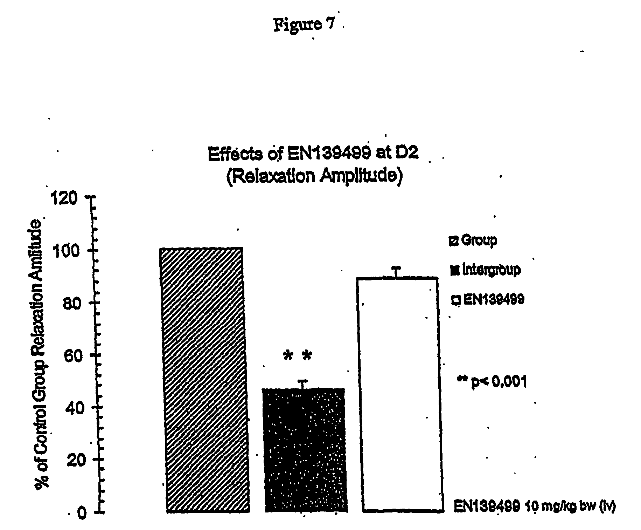

Figure 7 shows bar graphs of the effect on the amplitude of the relaxation component of gut motor activity recorded as in Figure 3, except at duodenal site D2 of anaesthetized rats treated with compound EN139499 (10 mg·kg⁻¹ body weight, intravenous administration). Amplitude of relaxation is expressed as percent of control "group" activity. Bars show the relative amplitudes of the relaxation component of motor activity for "group" motor activity prior to administration of EN139499 (diagonal bar, 100%); for "intergroup" motor activity prior to administration of EN139499 (filled bar); and for stimulation of activity after administration of EN139499 (open bar).

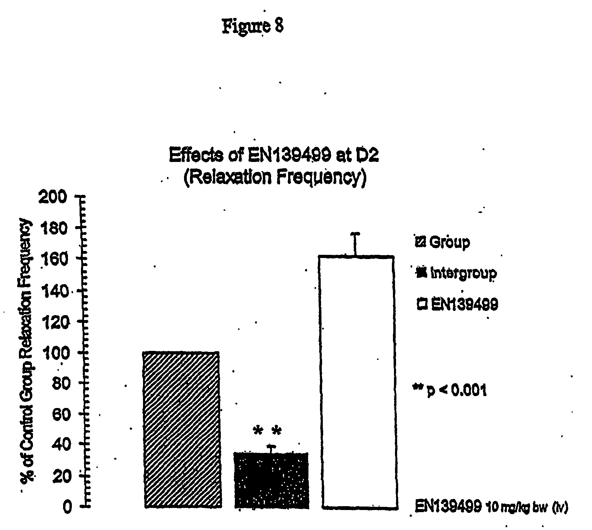

Figure 8 shows bar graphs of the effect on the frequency of the relaxation component of gut motor activity recorded as in Figure 4, except at duodenal site D2 of anaesthetized rats treated with compound EN139499 (10 mg·kg⁻¹ body weight, intravenous administration). Frequency of relaxation is expressed as percent of control "group" activity. Bars show the relative frequency of the relaxation component of motor activity for "group" motor activity prior to administration of EN139499 (diagonal bar, 100%); for "intergroup" motor activity prior to administration of EN139499 (filled bar); and for stimulation of activity after administration of EN139499 (open bar).

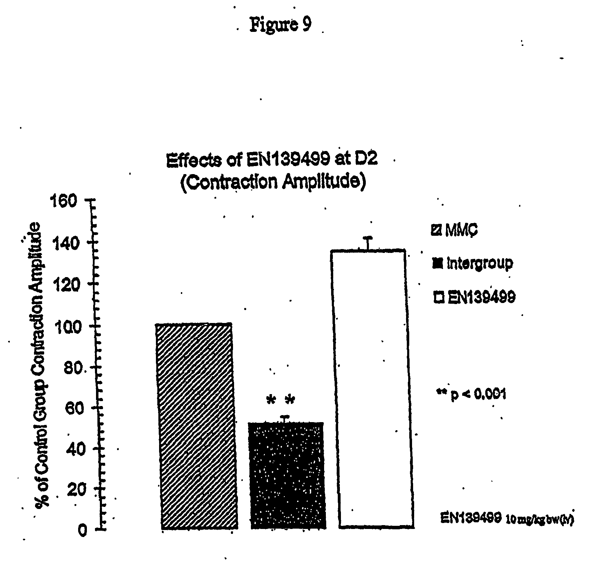

Figure 9 shows bar graphs of the effect on the amplitude of the contraction component of gut motor activity recorded as in Figure 5, except at duodenal site D2 of anaesthetized rats treated with compound EN139499 (10 mg·kg⁻¹ body weight, intravenous administration). Amplitude of contraction is expressed as percent of control "group" activity. Bars show the relative amplitude of the contraction component of motor activity for "group" motor activity prior to administration of EN139499 (diagonal bar, 100%); for "intergroup" motor activity prior to administration of EN139499 (filled bar); and for stimulation of activity after administration of EN139499 (open bar).

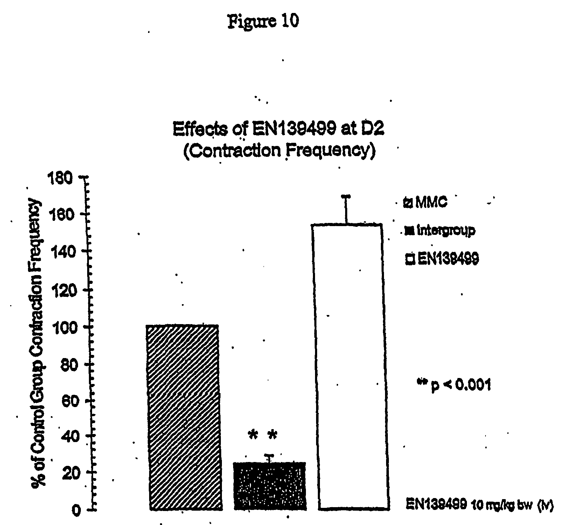

Figure 10 shows bar graphs of the effect on the frequency of the contraction component of gut motor activity recorded as in Figure 6, except at duodenal site D2 of anaesthetized rats treated with compound EN139499 (10 mg·kg⁻¹ body weight, intravenous administration). Frequency of contraction is expressed as percent of control "group" activity. Bars show the relative frequency of the contraction component of motor activity for "group" motor activity prior to administration of EN139499 (diagonal bar, 100%); for "intergroup" motor activity prior to administration of EN139499 (filled bar); and for stimulation of activity after administration of EN139499 (open bar).

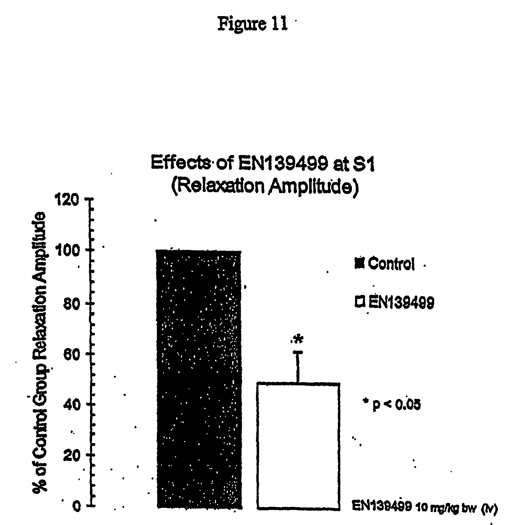

Figure 11 shows bar graphs of the effect on the amplitude of the relaxation component of gut motor activity recorded in the gastric antrum (site S1) of anaesthetized rats treated with compound EN139499 (10 mg·kg⁻¹ body weight, intravenous administration). Amplitude of relaxation is expressed as percent of control "group" activity. Bars show the relative amplitudes of the relaxation component of motor activity for "group" motor activity prior to administration of EN139499 (filled bar, 100%) and for inhibition of activity after administration of EN139499 (open bar).

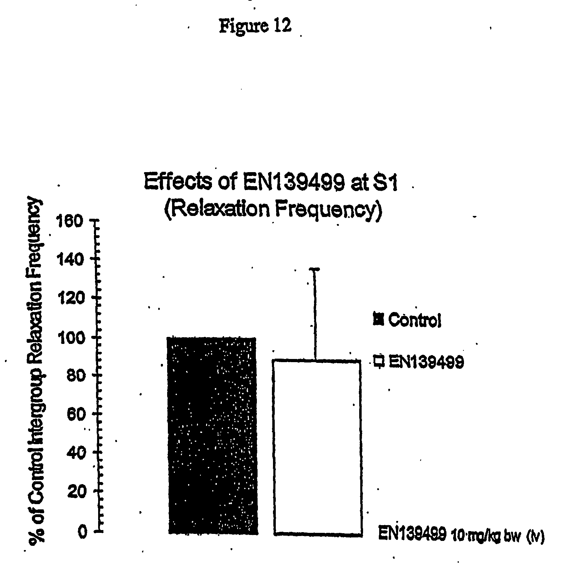

Figure 12 shows bar graphs of the effect on the frequency of the relaxation component of gut motor activity recorded in the gastric antrum (site S1) of anaesthetized rats treated with compound EN139499 (10 mg·kg⁻¹ body weight, intravenous administration). Frequency of relaxation is expressed as percent of control "group" activity. Bars show the relative frequency of the relaxation component of motor activity for "group" motor activity prior to administration of EN139499 (filled bar, 100%) and for inhibition of activity after administration of EN139499 (open bar).

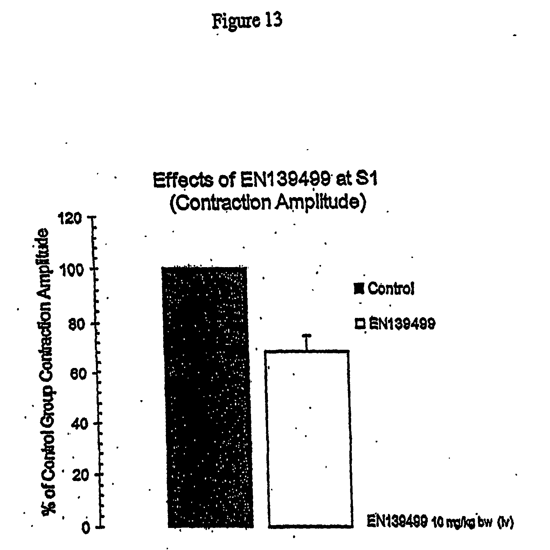

Figure 13 shows bar graphs of the effect on the amplitude of the contraction component of gut motor activity in the gastric antrum (site S1) of anaesthetized rats treated with compound EN139499 (10 mg·kg⁻¹ body weight, intravenous administration). Amplitude of contraction is expressed as percent of control "group" activity. Bars show the relative amplitude of the contraction component of motor activity for "group" motor activity prior to administration of EN139499 (filled bar, 100%) and for inhibition of activity after administration of EN139499 (open bar).

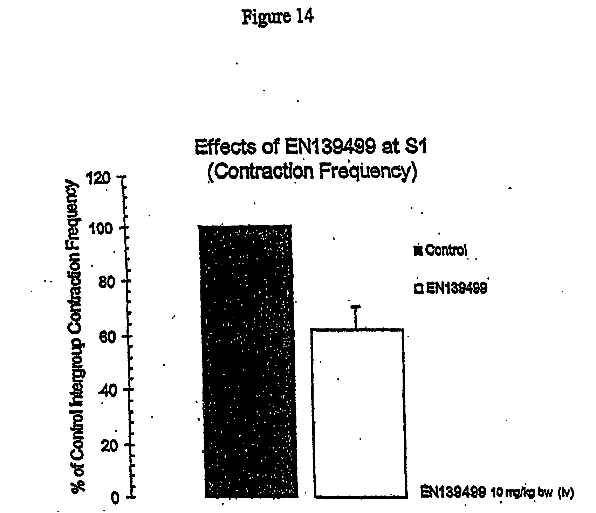

Figure 14 shows bar graphs of the effect on the frequency of the contraction component of gut motor activity in the gastric antrum (site S1) of anaesthetized rats treated with compound EN139499 (10 mg·kg⁻¹ body weight, intravenous administration). Frequency of contraction is expressed as percent of control "group" activity. Bars show the relative frequency of the contraction component of motor activity for "group" motor activity prior to administration of EN139499 (filled bar, 100%) and for inhibition of activity after administration of EN139499 (open bar).

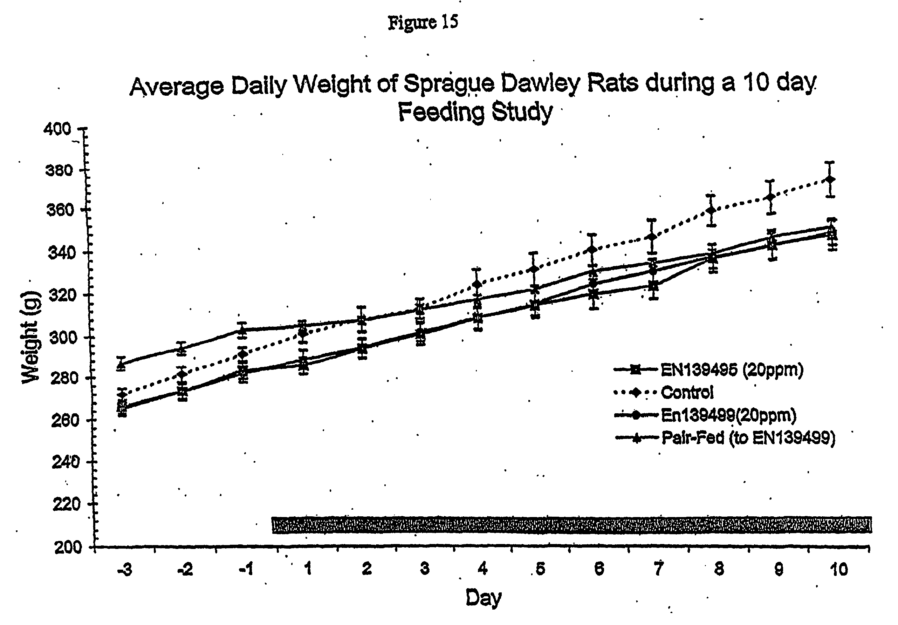

Figure 15 shows the average daily weight of male Sprague-Dawley rats during a 10 day feeding study, (days 1 to 10). Average daily weight expressed in grams (g) is shown for rats fed: normal powdered feed (positive control, diamonds); normal powdered feed supplemented with compound EN139495 (3-acetyl-DON carbonate, 20 ppm); normal powdered feed supplemented (mixed) with compound EN139499 (20 ppm); and normal powdered feed supplemented with EN139499 in a "pair-fed" quantity. See text for details. All rats were fed normal (unsupplemented) powdered food and monitored for three days (days -3 to -1) prior to commencement of study to ensure there were no significant baseline variations in food consumption and weight gain between the control and test groups.

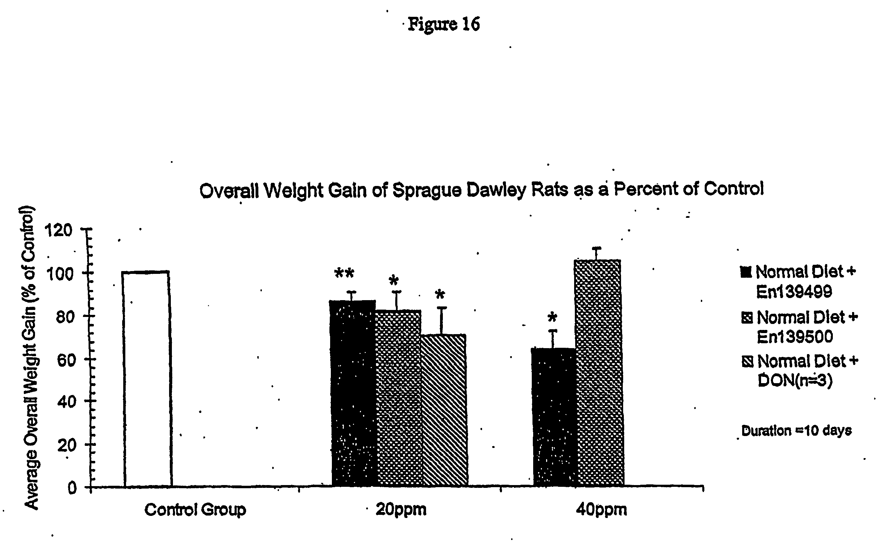

Figure 16 shows bar graphs of overall weight gain as percent of control in male Sprague-Dawley rats in a 10 day feeding study. Animals were fed normal powdered feed (normal diet) supplemented (mixed) with EN139499 (filled bars) at 20 ppm or at 40 ppm; normal diet supplemented with EN139500 (checkered bars) at 20 ppm or at 40 ppm; normal diet supplemented with DON (diagonal bars) at 20 ppm; or normal diet alone (control group, open bars).

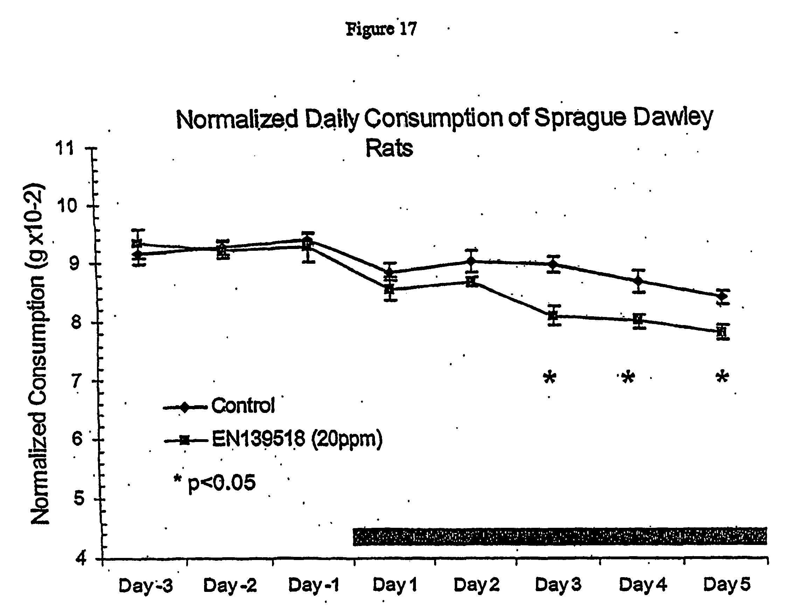

Figure 17 shows normalized daily consumption of feed (g x 10⁻²) by male Sprague-Dawley rats in a feeding study over time (days) of study. Animals were grouped by diet beginning at Day 1, when rats were switched from normal powdered feed (normal diet) to normal powdered feed mixed with EN139518 (rectangles) at 20 ppm or normal diet alone (control group, diamonds). Data points showing significant (p < 0.05) difference in consumption between normal control diet and EN139518 diet are indicated by asterisks (Days 3 to 5).

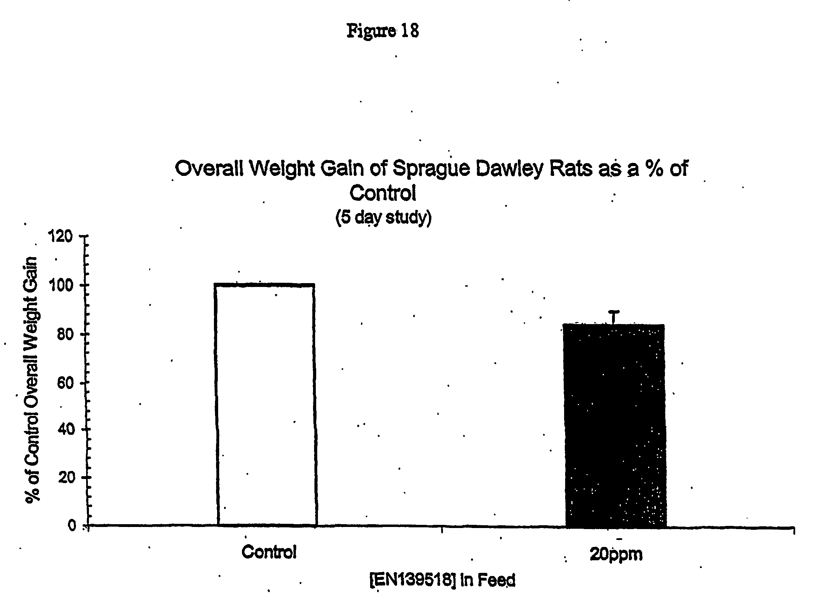

Figure 18 shows overall weight gain as percent of control group's overall weight gain in male Sprague-Dawley rats in 5 day feeding study. Rats fed normal diet (control group, open bar); rats fed normal diet mixed with EN139518 at 20 ppm (filled bar) as described for Figure 17.

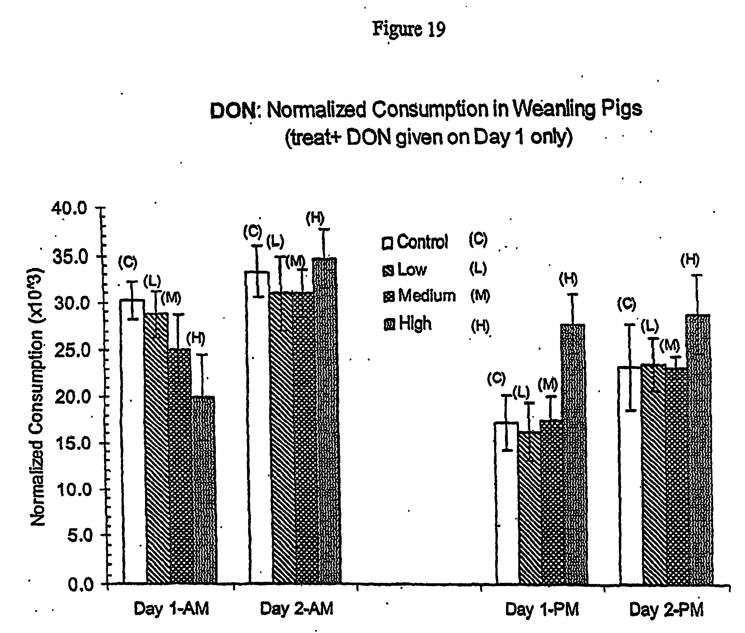

Figure 19 shows bar graphs of normalized consumption of food over two days (Day 1 and Day 2) at morning (AM) and evening (PM) meals by pigs that have ingested a single food treat containing DON at 0.11 mg/kg body weight (low dose, L), 0.17 mg/kg body weight (medium dose, M), or 0.34 mg/kg body weight (high dose, H) provided in the morning (AM) of Day 1, approximately 20 minutes prior to the morning meal. Control (C) animals received a food treat without DON at Day 1 AM.

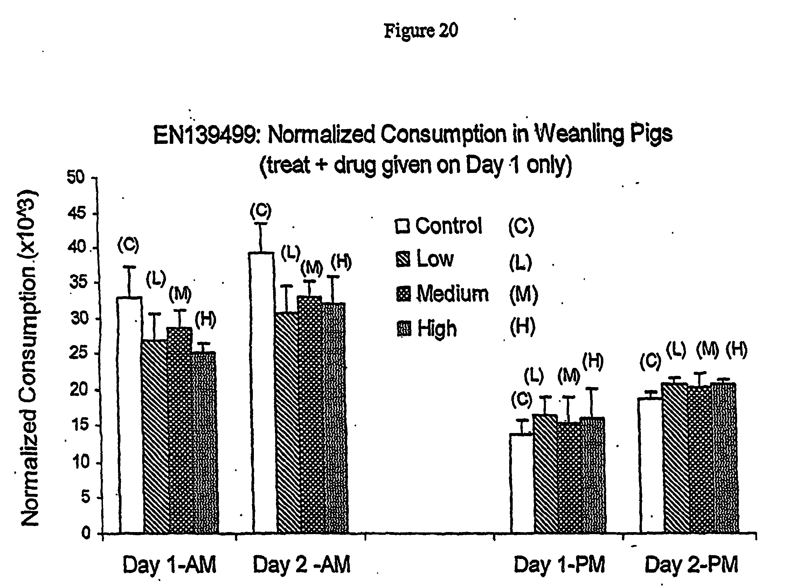

Figure 20 shows normalized consumption of food by pigs as in Figure 19, except that pigs received a food treat containing Compound EN139499 instead of DON. Control animals received a food treat without EN139499 at Day 1 AM.

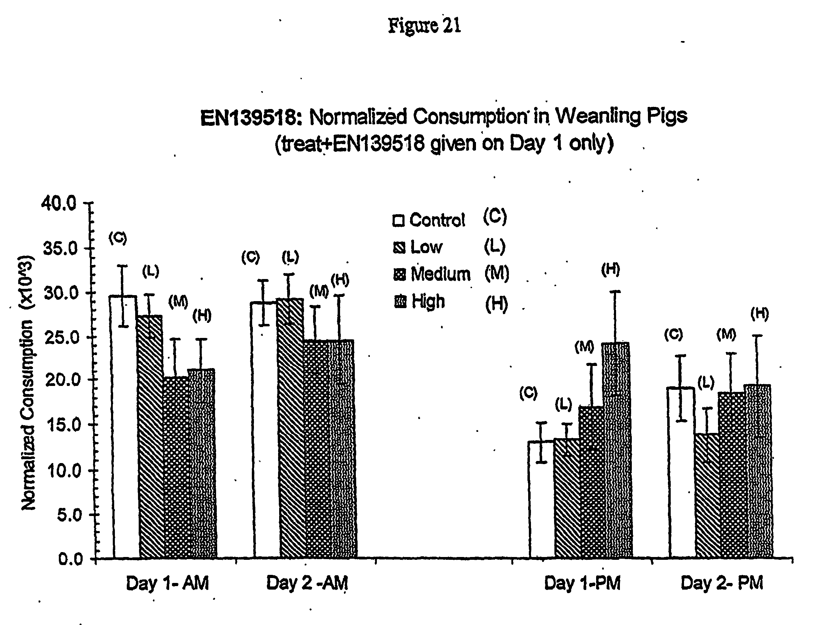

Figure 21 shows normalized consumption of food by pigs as in Figure 19, except that pigs received a food treat containing Compound EN139518 instead of DON. Control animals received a food treat without EN139518 at Day 1 AM.

### DETAILED DESCRIPTION

This invention provides pharmaceutical compositions and compounds and compositions for use in regulating food intake and treating excessive weight gain and obesity by modulating the motor activity of gut organs in humans and other vertebrate animals. The uses and compositions of the invention are based on the discovery that trichothecene compounds, such as the naturally occurring 4-deoxynivalenol (DON), stimulate the pattern of contractions and relaxations in organs of the gut that normally occur when food is ingested. Stimulation of this "fed pattern" of gut motility signals satiety, that is, the feeling of fullness, which is an important factor that affects the time that an individual spends eating. Trichothecenes, such as DON, act at a site outside the organs of the gut and send a signal, which is sent down neural pathways leading to the smooth muscle of the gut organs (see, international application No. PCT/CA00/00790, filed July 6,2000, incorporated herein by reference).

In addition, a number of studies have examined the ability of DON and related trichothecene mycotoxins to inhibit protein synthesis, e.g., as studied in cell cultures of Vero cells (green monkey kidney cells), murine erythroleukemia (MEL) cells, and rat spleen lymphocytes (see, e.g., Erhlich and Daigle, *Biochem*. *Biophys. Acta*, *923:* 206-213 (1987); Rotter et al., *J*. *Toxicol. Env. Health*, *48:* 1-34 (1996)). It appears that DON and related trichothecenes are able to bind the 60S subunit of the eukaryotic ribosome and interfere with the action of peptidyltransferase. Such toxicology studies have led to the conclusion that certain characteristic structural features of DON and related naturally occurring trichothecenes have been considered essential to their known toxic activities. In particular, the 9,10 double bond, the presence of a 12,13-epoxy ring, the 8-keto group, and the presence of hydroxyl or other groups on the trichothecene nucleus, as found in DON and related naturally occurring trichothecenes mycotoxins, have been regarded as critical to the toxicity of these mycotoxins, including the observed ability to reduce food intake in humans and to induce feed refusal in other animals that ingest trichothecenes (see, e.g., Betina, *Chem.-Biol. Interactions, 71:* 105-146 (1989)).

This invention provides new, multi-ring organic compounds that regulate gut motility. Especially useful are compounds described herein that stimulate the fed pattern of gut motility in humans and other vertebrate animals to signal satiety (fullness) and reduce food intake. Contrary to the established view regarding the importance of certain structural features to toxic effects of DON and other trichothecene mycotoxins based on inhibition of protein synthesis (see, above), the compounds of the invention include those in which the 9,10 double bond, and/or the 8-keto group, and/or the 3,4-epoxide ring normally found in known trichothecenes, such as DON, have be reduced or otherwise altered.

In order to more clearly describe the invention the following terms are defined:

As used herein, "gut" refers to the gastrointestinal tract consisting of the stomach, small intestine, and large intestine.

As used herein, "gut motility" or "gut motor activity" refers to the motor behavior of the smooth muscle in the gastrointestinal organs (stomach, small intestine, and large intestine) of humans and other animals which activity consists of periods of alternating muscular contractions and relaxations, as well as periods of quiescence or relatively little activity. For example, in normal, healthy humans and other animals, the frequency and amplitude of muscular contractions and relaxations of the small intestine become heightened when food is ingested in order to propel food aborally (forward) into the intestines for nutrient extraction and absorption (see, "fed pattern" of gut motility, below). Other patterns of gut motility may occur depending on the presence or absence of food in various parts of the gut organs (see below). Furthermore, measurements of motor activity have shown that the proximal portion of a particular gut organ may exhibit motor behavior that differs from the activity in a distal portion of the organ, such as in the case of the duodenum (the beginning portion of the small intestine) and the ileum (the terminal portion of the small intestine).

As used herein, "fed pattern" and "fed pattern activity" are synonymous and refer to the continuous pattern of contractions and relaxations of the small intestine of the gut in an animal, including humans, that normally occurs as the result of ingesting food. The fed pattern of gut motility propels ingested food through the gut for nutrient extraction and absorption, and eventually excretion of unabsorbed material as waste. The fed pattern of gut motor activity typically begins within minutes of ingesting food and is responsible for signaling satiety, that is, the feeling of fullness. Thus, satiety from the fed pattern of gut motility normally informs an individual that eating can be ended. Satiety is sensed by an individual via the fed pattern of gut motility long before the brain has an opportunity to analyze the nutrient content of the blood (a separate process that takes place hours after food has been consumed and that is responsible for signaling cravings for specific nutrients, such as, proteins, carbohydrates, salt, and fats, which are maintained at specific levels for health).

The fed pattern is both characteristic and different for each organ and even different sites in the same organ of the gut. In the small intestine, the fed pattern is characterized by a continuous series of contractions and relaxations of the smooth muscle, which mixes intestinal contents, propels food aborally into the intestines, and delays anterograde propulsion to enhance substrate absorption (Lundgren et al., *Dig. Dis Sci.,* 34: 264-283 (1989)). When measured and recorded *in vivo* by the method of Krantis et al. (*Can. J. Physiol. Pharmacol., 74:* 894-903 (1996)), the fed pattern of gut motor activity in the duodenum is a characteristic intense pattern of hyperactivity whereas simultaneously in the gastric antrum, the fed pattern is characterized as a measurable suppression or decrease in recorded tissue motor activity. This fed pattern activity replaces the "fasting pattern" of gut motor activity (see, below), which occurs after food has been propelled through the gut for nutrient extraction. Fed pattern motility is activated primarily by peripheral autonomic ganglia via primarily vagal inputs and also, but to a lesser extent, is controlled by the central nervous system (CNS) (see, Yoshida et al., *J. Pharmacol. Exp. Therap., 256:* 272-278 (1991); Tanaka et al., *J. Surg. Res*., *53:* 588-595 (1996); Chung et al., *Can. J. Physiol. Pharmacol., 70*: 1148-1153 (1992)). Over-activation of autonomic nerves accelerates the onset and increases the duration of the fed pattern, concurrently increasing the frequency and amplitude of propagatory motor activity of the gut (Hall et al., *Am. J. Physiol., 250:* G501-G510 (1986); Johnson et al., *Am. J. Surg.,167:* 80-88 (1994)). As noted above, trichothecenes, such as trichothecene mycotoxins, such as DON, and new compounds described herein can be used in proper amounts to stimulate the fed pattern of gut motility.

"Fasting pattern" or "fasting cyclic motor pattern" of gut motor activity refers to the motor behavior of the gut in the absence of ingested food matter or before ingesting food, when no ingested material is present for propulsion from the stomach and into intestines. In the duodenum (the start of the small intestine), the fasting pattern of gut motor activity is characterized by alternating periods of spontaneous, irregular contractions and relaxations (referred to as "group" or "MMC" activity) and relatively quiescent periods (referred to as "intergroup" activity). An example of a recording of duodenal fasting pattern with its alternating grouped and intergroup activities is shown in the early portion (between t = 8 and t = 40 minutes) of the recording of gut motility in Figure 2 prior to administration of compound EN139499. In the ileum (the end region of the small intestine), the fasting pattern is characterized by random contractile and/or relaxant motor activity or a generally quiescent state. Ingestion of food matter interrupts the fasting pattern of gut motility and stimulates the continuous activity of the fed pattern of gut motility.

Until recently, methods were not available for accurately recording, measuring, and characterizing gut motility in that only one component, either contraction or relaxation, could be measured under experimental conditions. More recently, however, Krantis and co-workers have developed a method of simultaneously measuring the contraction and relaxation components of gut motility for various organs of the gastrointestinal tract using miniaturized, flexible, foil strain gauges that can be attached *in vivo* to various locations on organs of the gut (see, Krantis et al., *Can*. *J*. *Physiol*. *Pharmacol,* 74: 894-903 (1996)). In this method, wires from the gauges attached to the organs are connected to a computerized data analysis system. The method of Krantis et al. (1996) may be used for pharmacological, neurological, and physiological studies of the gut *using in vivo, ex vivo* (organs positioned out of the body cavity), and *in vitro* (extricated tissue from gut organs) procedures. The ability to simultaneously record contractions and relaxations in gut organs and at multiple sites within an organ provides a more precise characterization of gut motility, including distinct patterns of gut motility (i.e., fasting and fed patterns), and the effect of food and various chemical compounds on such patterns.

In the fasted state, the gut exhibits a cyclic motor behavior known as "group", "MMC", "migrating motor complex", or "migrating myoelectric complex". MMCs are associated with interdigestive propulsion of intestinal contents and involve sequential activation of excitatory and inhibitory neurons to propagate cycles of contractions and relaxations that originate in the stomach and terminate at the ileum. An MMC cycle consists of three distinct phases: phase I is a quiescent phase; phase II is a period of irregular spiking of activity, and phase III is a short period of rapid spike bursts of activity. MMCs provide a basic intrinsic motor pattern, which functions as a "housekeeper" of the small intestine. For example, the highly propulsive phase III motor activity of each MMC cycle sweeps the intestinal lumen, clearing it of remnants to prevent bacterial overgrowth, back flow, and the accumulation of intestinal secretions (Caenepeel et al., *Dig. Dis. Sci*., *34*: 1180-1184 (1989)). Using the method of Krantis et al. (1996), it is now clear that gut motility may comprise both contractions and relaxations of smooth muscle. In the absence of food, the "group" activity of the fasting pattern of intestinal gut motility appears to correspond to the same type of motor activity classically ascribed to phase III of the classic MMC cycle. The presence of food in the intestinal lumen induces a switch from the fasting pattern to the fed pattern of gut motor activity.

The method of Krantis et al. (1996) has also enabled the discovery of the mode of action of trichothecenes on gut motility. The trichothecene 4-deoxynivalenol (DON) acts at sites outside the gut to stimulate the fed pattern of gut motility, which characteristically occurs after ingesting food and which signals satiety, that is, the sensation of fullness (see, international PCT application No. PCT/CA00/00790). Such findings explain the well-documented anorectic or feed refusal behavior of humans and other animals that have ingested crops contaminated with fungal species that produce DON or other trichothecenes.

Furthermore, the time course and profile of action of DON and the compounds of the invention for altering gut motor activity as determined by the method of Krantis et al. (1996) is not consistent with the time course and profile for trichothecene dependent inhibition of protein synthesis or hormone based-regulation of food intake (see, below), as such routes of action would be expected to require significantly longer periods of time prior to seeing an effect on gut motility and/or food intake.

Historically, trichothecene compounds were identified as one of the toxic secondary metabolites produced by various fungi that can contaminate crops, hence the designation trichothecene mycotoxins. Animals, including humans, that ingest such contaminated crops may experience a variety of pathological symptoms of mycotoxicosis, such as vomiting, diarrhea, hemorrhagic lesions in internal organs, alimentary toxic aleukia (ATA), agranolocytosis, aplastic anemia, necrotic angina, inflammation of mucous membranes, refusal to eat, convulsions, sepsis, and in some cases, death (see, for example, Ueno, "Trichothecene Mycotoxins: Mycology, Chemistry, and Toxicology," in *Advances in Nutritional Research 1980, 3:* 301-353 (1980)).

As used herein, "trichothecene mycotoxin" or "trichothecene" refers to a member of a group of sesquiterpenoid chemical compounds based on the non-olefinic parent or core compound trichothecane. All trichothecenes are modified sesquiterpenes, and contain an olefinic (double) bond (hence, trichothec*ene*) between carbon atoms at positions 9 and 10 (C-9, C-10), and an epoxy ring formed between carbon atoms at positions 12 and 13 (C-12, C-13). Thus, trichothecenes are also characterized as 12,13-epoxytrichothecene compounds. Ueno classified naturally occurring trichothecene mycotoxins into four groups based on structural and also fungal characteristics (see, for example, Ueno, 1980, *supra).* According to this classification scheme, members of a group of trichothecenes represented by nivalenol are non-macrocyclic compounds that have the carbon-8 (C-8) substituted with a ketone (oxo-) group. In addition to nivalenol, the group of "nivalenol-related" trichothecenes includes such naturally-occurring trichothecene mycotoxins as 4-deoxynivalenol (DON), trichothecolon, trichothecin, 3-acetyldeoxynivalenol (3-acetyl-DON), 7-acetyldeosynivalenol, 3,15-diacetyldeoxynivalenol, 4-acetylnivalenol (fusarenon-X), and 4,15-diacetylnivalenol. As used herein, "DON", "4-DON", "deoxynivalenol", "4-deoxynivalenol", and "vomitoxin" all refer to the same trichothecene compound having the chemical structure shown in Fig. 3. Thus, nivalenol differs from DON in that nivalenol contains a hydroxyl group at C-4, whereas DON lacks the hydroxyl group ("4-deoxy") at position 4.

Although clearly capable of causing severe and widespread incidences of toxicosis when ingested in sufficiently high quantities, DON is nevertheless considered as one of the least potent trichothecenes with respect to sub-lethal toxicosis (see, for example, Prelusky et al., *Arch. Environ. Contam. Toxicol., 22:* 36-40 (1992); Friend et al., *Can. J. Anim. Sci., 66:* 765-775 (1986); Ueno, in Developments in Food Science IV, Trichothecenes, chemical, biological, and toxicological aspects (Elsevier, Amsterdam, 1983), pp. 135-146).

DON appears to undergo no extensive liver metabolism and is readily and predominantly eliminated in the urine.

Trichothecenes used in the synthesis of compounds of the invention may be produced biologically from fungal cultures or by chemical synthesis. Some are available commercially. A variety of soil fungi that have been found contaminating and growing on cereal grains and other crops produce trichothecenes as secondary metabolites. Such fungi include species of *Fusarium*, *Tricothecium, Trichoderma*, *Myrothecium, Cylindrocarpon,* and *Stachybotrys* (see, Ueno, 1980). Methods of producing and purifying DON and acetyl esters of DON (such as 3-acetyl DON and 15-acetyl DON) from *Fusarium* cultures have been described (see, *Can. J. Microbiol., 29:* 1171-1178 (1983); Miller and Blackwell, *Can. J. Bot., 64:1-5* (1986); Greenhalgh et al., *Proceedings of the 6th IUPAC International Symposium on Mycotoxins and Phycotoxins:* 137-152 (Steyn. P.S., ed.) (Elsevier Press, Amsterdam, 1986); Miller and Amison *(Can. J. Plant Path., 8*: 147-150 (1986)). Thus, various trichothecenes such as DON and 3-acetyl-DON that are used in the synthesis of compounds described herein may be produced and extracted from fungal cultures using standard culture and production techniques (see, for example, Ehrlich et al., *Biochim. Biophys. Acta. 932:* 206-213 (1987); Ueno, 1980 *(supra)* and references cited therein). For example, 3-acetyl DON is readily purified from fermentations of *Fusarium spp.* cultures. DON can then be obtained from the 3-acetyl DON by saponification. As noted above, DON is an abundant, natural contaminant of com and wheat. Thus, DON and other trichothecenes may also be isolated from contaminated crops. Alternatively, they can be isolated from the Brazilian shrubs *Baccharis magapotomica* and *cordfolia* (Kupchan et al. *J. Org. Chem., 42*: 4221-4225 (1977)).

As described in more detail below, this invention provides compounds for use in treating excessive weight gain and obesity taking advantage of the ability of new compounds that act like known trichothecene compounds to induce the fed pattern of gut motility and satiety and, thereby, reduce or halt food intake. Induced cessation of eating in non-human animals is also referred to as "feed refusal". Uses of compounds in treating excessive weight gain and obesity described herein comprise administering to an individual a compound described herein, which stimulates the fed pattern of gut motility and, thereby, satiety. Sensing fullness, the individual is thus given a signal to stop eating. When circulating levels of the administered compound decrease, satiety will decline, and the individual may continue to eat or feed.

### Compounds Useful in the Invention

The chemistry of naturally-occurring trichothecenes is well known (see, for example, Ueno, "Trichothecene Mycotoxins: Mycology, Chemistry, and Toxicology," in *Advances in Nutritional Research 1980, 3:* 301-353 (1980); Williams, *Arch. Environ Contam. Toxicol, 18:* 374-387 (1989)). Accordingly, once the structure of a new compound described herein is known, the compound may be synthesized from certain trichothecenes, such as 3-acetyl DON, or various synthetic derivatives thereof, by organic synthesis (see Examples below). The structures of compounds described herein are readily determined using standard methods available in the art, many of which have been used to determine or confirm structures of known trichothecenes. Especially useful for determining or confirming structures of compounds of the invention are nuclear magnetic resonance (NMR) analysis, thin-layer chromatography (TLC), and mass spectroscopy (MS).

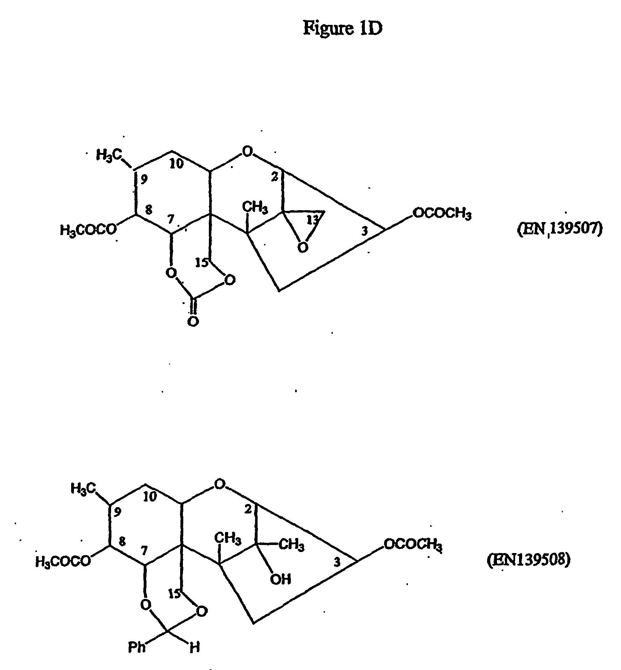

Certain pharmaceutical compositions of the invention comprise a gut motility regulating "8-O-substituted-7α,15-carbonate compound" of formula I, as shown below (including selected trichothecene-type numbering for various atoms and groups in the chemical structure): 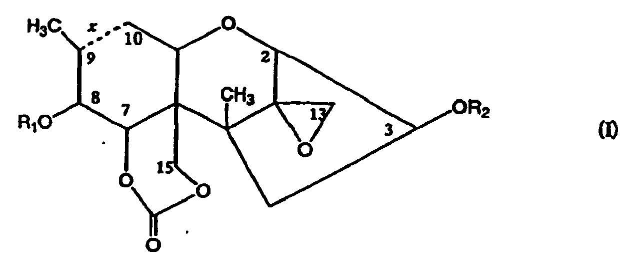 wherein, *x* is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; or
   a 5- or 6-membered heteroaromatic ring substituted with a substituent
   selected from the group consisting of halogen, alkyl (for example, methyl), alkoxy (for example, methoxy), thiomethyl, sulfinylmethyl, sulfonylmethyl, and combinations thereof.
Representative species of formula I include 3α-acetoxy-12,13-epoxy-8α-hydroxytrichothec-9-en-7α,15-carbonate (designated EN139499, see Figure 1A); 3α,8α-diacetoxy-12,13-epoxytrichothec-9-en-7α,15-carbonate (designated EN139500, see Figure 1A); and 3α,8α-diacetoxy-12,13-epoxy-9α-methyltrichothacan-7α,15-carbonate (designated EN139507) (see, Figure 1D).

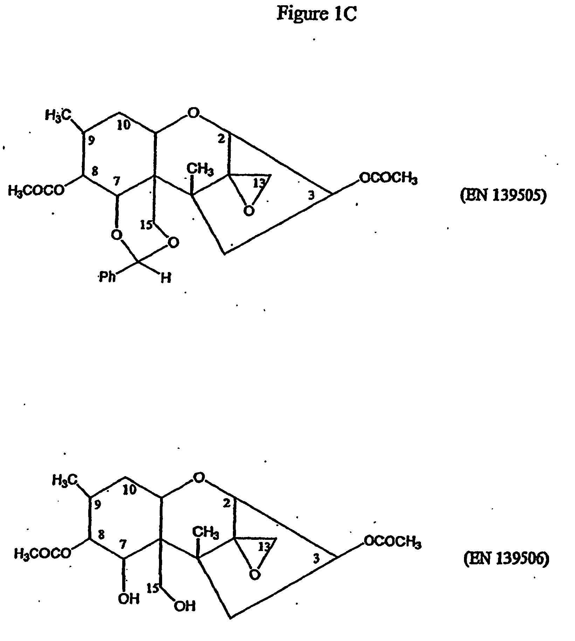

Other pharmaceutical compositions of the invention comprise a gut motility regulating "acetal compound" of formula II, as shown below (including selected trichothecene-type numbering for various atoms and groups in the chemical structure): 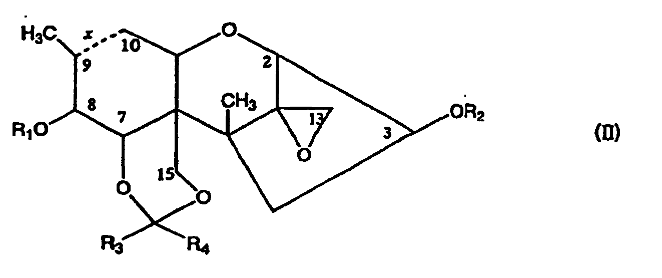 wherein x is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; or
   a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof;
R₃ and R₄ are, independently:
a hydrogen;
a C₁ to C₆ alkyl;
a phenyl group;
a phenyl group substituted with a substituent selected from the group consisting of halogen, alkyl (for example, methyl), alkoxy (for example, methoxy),
thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Examples of representative species of formula II include 3α-acetoxy-7α,15-benzylidene-12,13-epoxy-9α-methyltrichothecan-8α-ol (designated EN139501, see Figure 1A) and 7α,15-benzylidene-3α,8α-diacetoxy-12,13-epoxy-9α-methyltrichothecane (designated EN139505) (see, Figure 1C).

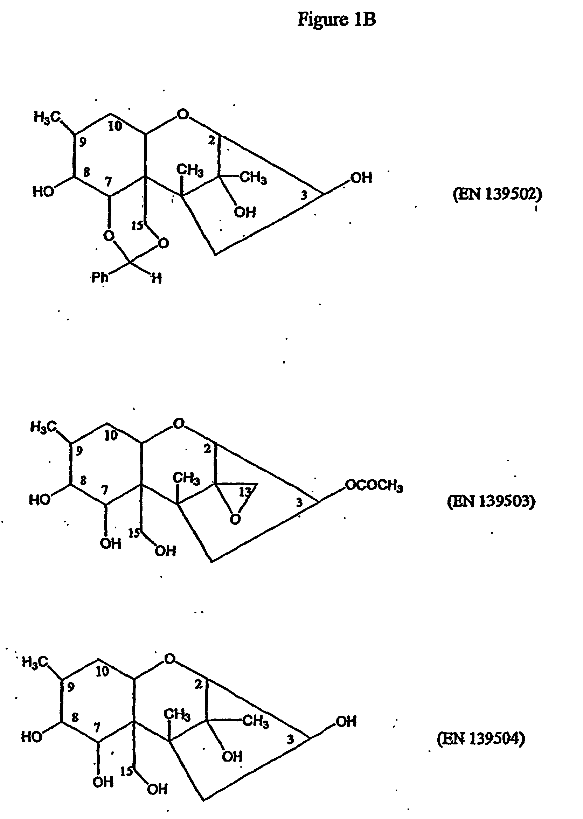

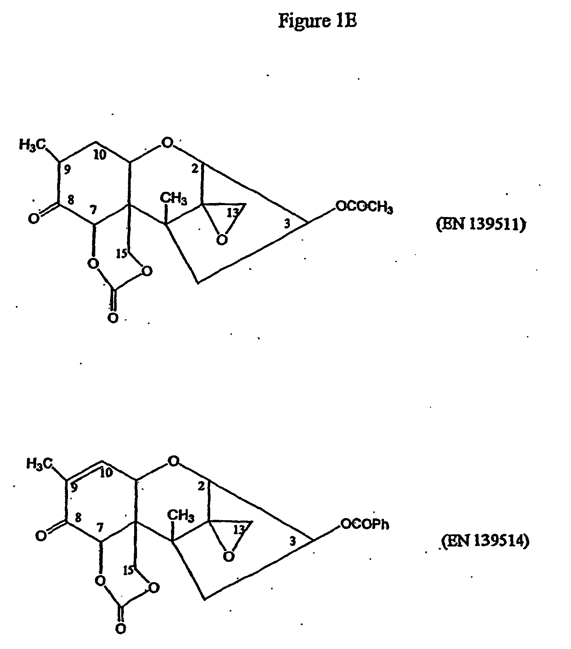

The invention also provides pharmaceutical compositions that comprise a gut motility regulating "7α, 15-diol" compound of formula III, as shown below (including selected trichothecene-type numbering for various atoms and groups in the chemical structure): 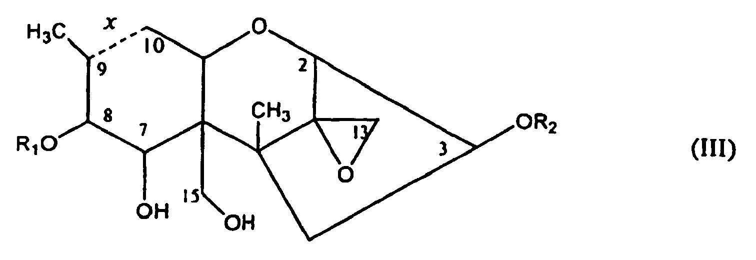 wherein x is a single or double bond between carbon atom-9 and carbon atom- 10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfunylmethyl, and combinations thereof;
   a 5- or 6-membered heteroaromatic ring;
   a 5- or 6-membered heteroaromatic ring substituted with a substituent selected from the group consisting of halogen, alkyl (for example, methyl), alkoxy (for examle, methoxy), thiomethyl, sulfinylmethyl, sulfonylmethyl, and combinations thereof.
   Representative species of formula III include 3α-acetoxy-12,13-epoxy-9α-methyltrichothecan-7α,8α,15-triol (designated EN139503, see Figure 1B) and 3α,8α-diacetoxy-12,13-epoxy-9α-methyltrichothecan-7α,15-diol (designated EN139506, see Figure 1C).

The invention also provides pharmaceutical compositions that comprise a gut motility regulating 8-keto-7α,15-carbonate compound of formula IV, as shown below (including numbering for various atoms and groups in the chemical structure): 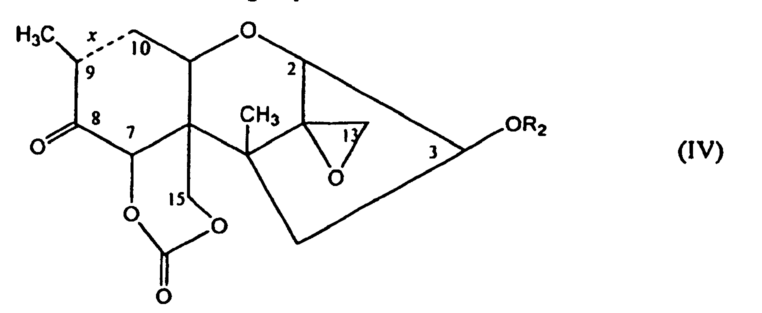 wherein x is a single or a double bond between carbon atom-9 and carbon atom-10;
R₂ is:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and
   a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof; with the proviso that when *x* is a double bond, then R₂ is not hydrogen or C(O)R₅ wherein R₅ is methyl.
Preferred representative species of formula 1V include 3α-acetoxy-12, 13-epoxy-9α-methyltrichothecan-8-on-7α,15-carbonate (designated EN 139511, see Figure 1E) and 3α-benzoyloxy-12,13-epoxytrichothec-9-en-8-on-7α,15 carbonate (designated EN139514. see Figure 1E).

The invention also provides pharmaceutical compositions that comprise a gut motility regulating regulating 8-keto-12,13-epoxy-compound of formula V, as shown below: 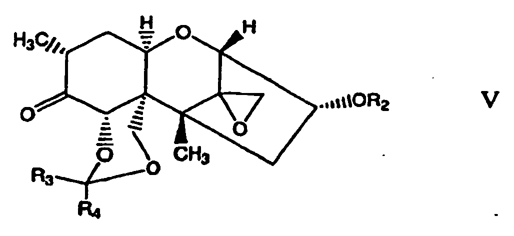 wherein R₂ is:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and
a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof; and
R₃ and R₄ are, independently:
a hydrogen;
a C₁ to C₆ alkyl;
a phenyl group;
a phenyl group substituted with a substituent selected from the group consisting of halogen, alkyl (for example, methyl), alkoxy (for example, methoxy), thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Preferred representative species of formula V include, without limitation, 3-α-acetoxy-7α,15-beazylidene-12,13-epoxytrichothecan-8-one (designated EN139519) and 7α, 15-benzylidene-12,13-epoxy-3β-bydroxytrichothecan-8-one (designated EN139520).

The invention also provides pharmaceutical compositions that comprise a gut motility regulating 8-keto-12α-hydroxy compound of formula VI as shown below: 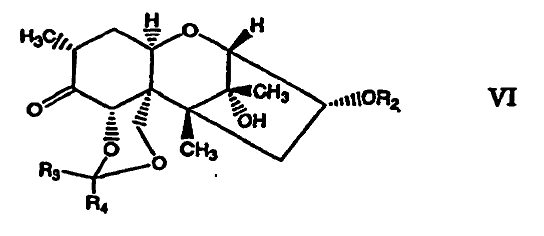 wherein R₂ is:
a hydrogen atom;
a C₁ to C₆ alkyl;
a C₁ to C₆ arylalkyl; or
an acyl group C(O)R₅, wherein R₅ is selected from the group consisting of:
   a C₁ to C₆ alkyl;
   a C₂ to C₆ alkenyl;
   a C₂ to C₆ alkynyl;
   a phenyl group;
   a phenyl group substituted with a substituent selected from the group consisting of halogen, alkyl (for example, methyl), alkoxy (for example, methoxy), thiomethyl, sulfinylmethyl, sulfonylmethyl, and combinations thereof;
   a 5- or 6-membered heteroaromatic ring; and
a 5- or 6-membered heteroaromatic ring substituted with a substituent selected from the group consisting of halogen, alkyl (for example, methyl), alkoxy (for example, methoxy), thiomethyl, sulfinylmethyl, sulfonylmethyl, and combinations thereof; and
R₃ and R₄ are, independently:
a hydrogen;
a C₁ to C₆ alkyl;
a phenyl group;
a phenyl group substituted with a substituent selected from the group consisting of halogen, alkyl (for example, methyl), alkoxy (for example, methoxy), thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.
Preferred representative species of formula VI include, without limitation, 7α,15-benzylidene-9α,12β-dimethyl-3α,12α-dihydroxytrichothecan-8-one (designated EN 139522).

Another compound that is useful in the compositions and uses of the invention is 12,13-epoxytrichothec-9-ene-3α,7α,8α,15-tetraol (designated EN139518).

The compound 7α, 15-benzylidene-9α,12β-dimethyltrichothecan-3α,8α,12α-triol (EN139502, Figure 1B) lacks the characteristic 9,10 double bond, 8-keto group, as well as 12,13-epoxide ring of trichothecenes. EN139502 does not induce the fed pattern of gut motility, but rather intensifies or enhances the fasting pattern of gut motility.

The invention also provides 7α,15-benzylidene-3α,8α-diacetoxy-9α,12β-dimethyltrichothecan-12α-ol (designated EN139508, Figure 1D).
The invention also provides a compound of formula (I), formula (II), formula (III), formula (IV), formula (V), or formula (VI) as defined above with the proviso that a compound of formula (III) as defined above is not the compound wherein simultaneously x is a double bond, R₁ is hydrogen and R₂ is C(O)R₅ wherein R₅ is methyl, or the compound wherein simultaneously x is a double bond, R₁ is hydrogen and R₂ is hydrogen, or the compound 12,13-epoxytrichothec-9-ene-3α, 7α, 8α, 15-tetra ol (designated EN 1395 18).
The invention also provides a compound of formula (I), formula (II), formula (III), formula (IV), formula (V), or formula (VI) as defined above for use as a medicament.

### Gut Motility Regulating Activity of Compounds

Some of the compounds of the invention that are useful in the pharmaceutical compositions and uses of the invention induce the fed pattern of gut motility, as determined using the method of Krantis et al. (1996), or a comparable method. Induction of fed pattern of gut motility in an individual signals satiety and reduces food intake (food uptake, eating, feeding), which is a desirable effect for treating excessive weight gain and obesity. In contrast, certain other compounds described herein, such as EN139502, lack the characteristic 12,13-epoxide ring of known trichothecenes and do not induce the fed pattern of gut motor activity. In fact, EN139502 is an example of a compound that does not induce fed pattern, but rather appears to enhance or intensify the fasting pattern of gut motor activity.

The method of Krantis et al. (1996) typically tests and analyzes a compound's ability to regulate gut motility in anaesthetized rat or piglet, though other animals may be used. Briefly, each animal is anesthetized (for example, with halothane:oxygen mixture) and compound or drug administered through a cannulated femoral artery. The animal is subjected to laparotomy, and foil strain gauges (Showa type N11, Durham Instruments, Pickering, Ontario) are affixed to selected sites on the gastrointestinal serosa using a tissue glue. Such sites typically include the gastric antrum ("S1" site), duodenum ("D1" and "D2" sites), and distal ileum. Recording gut motility using these three sites (i.e., S1, D1, and D2) provides the most comprehensive picture of gut motility and whether the fed pattern has been induced. Lead wires from each foil strain gauge are exteriorized and attached to a computerized data acquisition system for continuous, real-time recording of gut motility.

The activity of compounds can be further tested in feeding studies in which food consumption and weight gain are closely monitored in test subjects which receive various doses of a gut motility regulating compound described herein. Such studies may initially be carried out using small mammals, such as experimental rodents and pigs.

### Methods of Treatment, Pharmaceutical Compositions, Modes of Administration

The invention provides pharmaceutical compositions, which may be used in regulating food intake and treating obesity in humans and other animals. Compositions of this invention may also be particularly formulated for administering to non-human animals to control food intake and weight gain.

Other compositions of the invention comprise a compound that enhances or intensifies the fasting pattern of gut motility. Such compositions may be used to increase food uptake or to counteract the effect of compositions that induce fed pattern and, thereby, reduce food uptake. Compositions that increase food uptake are particularly useful to increase weight in animals used for food as in preparing commercial poultry and livestock for market. Other compositions may be appropriately formulated for administration to humans to stimulate food intake, for example, to treat malnutrition and anorexia in humans.

Humans and other vertebrate animals have the same basic gut neurophysiology with respect to controlling gut motility. Accordingly, animals that can be treated using the methods and compositions described herein include, without limitation, humans and other primates, swine, cattle, sheep, birds (poultry and other birds), horses, cats, dogs, and rodents, including hamsters, guinea pigs, rats, and mice. Both pharmaceutical compositions and compositions for administration to other animals of the invention comprise an effective amount of a compound described herein to achieve the desired effect on gut motility (fed or fasting pattern), to signal satiety to reduce food intake and thereby treat obesity or excessive weight gain, or to increase food intake to achieve increased nutrition or weight gain, all without significant or undesirable side effects, such as vomiting.

In one embodiment of the invention, a compound of the invention is administered, preferably orally, to an individual (human or other animal) prior to mealtime (e.g., without limitation 10, 20, or 60 minutes earlier) so that the compound regulates gut motility to effect a decrease food intake (or increase food intake depending on the activity of a particular compound) by the individual during the meal. In a particularly preferred embodiment, the effect of the compound wears off within one or several hours so as not to affect food intake during a subsequent meal, unless administered again to the individual.

Trained healthcare professionals are capable of assessing whether a human or other animal is clinically overweight, obese, underweight, and whether an individual is a candidate for treatment using the compositions of the invention. According to the invention, such conditions are treated by administering to a human or other animal a composition comprising one or more compounds described herein in an effective amount to regulate the pattern of gut motility and, thereby, either to decrease food intake or to increase eating time and food intake. Such compositions of the invention typically also comprise at least one pharmaceutically acceptable carrier (or an acceptable equivalent for use in non-human animals), which may be a liquid or a solid as described below.

Compositions of the invention may be in any of a variety of forms particularly suited for the intended mode of administration, including solid, semi-solid or liquid dosage forms, for example, tablets, lozenges, pills, capsules, powders, suppositories, liquids, powders, aqueous or oily suspensions, syrups, elixirs, and aqueous solutions. Preferably, the pharmaceutical composition is in a unit dosage form suitable for single administration of a precise dosage, which may be a fraction or multiple of a dose which is calculated to produce the desired affect on gut motility. The compositions will include, as noted above, an effective amount of the selected compound in combination with a pharmaceutically acceptable carrier or buffer, and, in addition, may include other medicinal agents or pharmaceutical agents, carriers, diluents, fillers and formulation adjuvants, or combinations thereof, which are non-toxic, inert, and pharmaceutically acceptable. In liquid mixtures or preparations, a pharmaceutically acceptable carrier is a buffer solution, such as a phosphate buffered saline or another pharmaceutically acceptable, especially isotonic, aqueous buffer. By "pharmaceutically acceptable" is meant a material that is not biologically, chemically, or in any, other way, incompatible with body chemistry and metabolism and also does not adversely affect any other component that may be present in the pharmaceutical composition.

For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like. Pharmaceutically acceptable liquid compositions can, for example, be prepared by dissolving or dispersing an active compound that regulates gut motility as described herein and optimal pharmaceutical adjuvants in an excipient, such as, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, triethanolamine oleate.

Standard methods of preparing dosage forms are known, or will be apparent, to those skilled in this art (see, for example, Remington's Pharmaceutical Sciences (Martin, E.W. (ed.) latest edition Mack Publishing Co., Easton, PA).

The primary active ingredient of a composition of this invention is one or more of the gut motility regulating compounds described herein. Such compounds may exert their activity on gut motility when ingested. Accordingly, preferred compositions of this invention are formulated for oral administration. Such compounds may also be administered parenterally, for example, by intravenous, intramuscular, or intraperitoneal injection.

For oral administration, which is preferred, compositions of the invention may be formulated as fine powders or granules containing of a compound described herein that affects gut motility and may also contain diluting, dispersing, and/or surface active agents. Compositions for oral administration may also be presented in water or in a syrup as a solution or suspension, in pills, tablets, capsules or sachets in the dry state, or in a nonaqueous solution or suspension wherein suspending agents may be included. Binders and lubricants may also be used in compositions for oral administration. Where desirable or necessary, flavoring, preserving, suspending, thickening, or emulsifying agents may be included. Tablets and granules are preferred oral administration forms, and these may be coated.

Parenteral administration, if used, is generally a method of injection. Injectable preparations can be prepared in conventional forms, either liquid solutions or suspensions, solid forms suitable for solution or suspension in liquid prior to injection, or as emulsions. For most purposes, a compound useful in regulating gut motility may be injected intravenously in a pharmaceutically acceptable buffer. However, it is within the scope of this invention that such a compound may alternatively be prepared as a bolus, which may contain a mordant for gradual release from an injection site. One approach for parenteral administration involves use of a slow release or sustained release system, such that a constant level of dosage is maintained (see, for example, U.S. Patent No. 3,710,795).

The exact, effective amount of a compound useful in regulating gut motility in the compositions and uses described herein will vary from subject to subject, depending on the age, weight and general condition of the subject, the degree of excessive weight gain or obesity being treated, the particular compound(s) used, the mode of administration, and the like. Thus, it is not possible to specify an exact amount for an ideal dose applicable to all individuals. However, it is expected that generally a compound of the invention will be used or tested in a range of 0.01 - 100 mg/kg body weight. Furthermore, the preferred useful dosage selected for a particular individual will be a sub-emetic dose, that is, a dose that does not evoke vomiting in the individual. For commercial pharmaceutical compositions, it is understood that a pharmaceutically effective and suitable amount of compound described herein will be determined, in the case of human use, by the healthcare professional in studies acceptable to the standards of the United States Food and Drug Administration (or comparable agency). For use in animals, an appropriate composition comprising a compound described herein will be determined and formulated according to the standards and practices for commercial animal feed or veterinary medicine.

Additional embodiments and features of the invention will be apparent from the following non-limiting examples.

### EXAMPLES

### Example 1. Starting compounds and assays.

The synthesis of various representative gut motility regulating compounds of the invention utilized a number of starting compounds that may be obtained commercially or using methods known in the art as indicated below.

Deoxynivalenol (3α,7α,15-trihydroxy-12,13-epoxy-trichothec-9-en-8-one, C₁₅H₂₀O₆, "DON") was produced biosynthetically by fungal cultures by Dr. David Miller (Department of Chemistry, Carleton University, Ottawa, Ontario, Canada). DON was purified from fermentation media from the fungal cultures basically according to the methods of Miller et al. (see, *Can. J. Microbiol., 29*:1171-1178 (1983); Miller and Blackwell, *Can. J. Bot., 64:* 1-5 (1986); Miller and Arnison (*Can. J. Plant Path., 8:147-*150 (1986)).

An acetyl ester of DON, 3α-acetoxy-12,13-epoxytrichothec-9-en-8-one (C₁₇H₂₂O₇, also called "3-acetyl DON") was produced and purified from fungal fermentations basically as described by Miller and Blackwell (*Can*. *J*. *Botany*, 64: 1-5 (1986)). Briefly, the fermentation broth from fungal cultures of *Fusarium culmorum* HLX1503 was filtered to remove mycelium and carefully neutralized to pH 7 with potassium carbonate. Sodium chloride (145 g) was added to each 1.5 liters of filtrate. The resultant solution was extracted four times with 500 ml of dichloromethane. The combined organic extracts were dried with anhydrous magnesium sulfate, and the solvent was evaporated under vacuum. The resulting crude material was chromatographed on 100 grams of silica gel. 3-acetyl DON was eluted from the silica gel with a mixture of ethyl acetate:hexane (6:4 mixture). One representative run of this purification procedure yielded 570 mg of 3-acetyl DON.

In addition as serving as the starting compound for the synthesis of a number of new compounds, 3-acetyl DON could also be converted to DON by saponification with an alkali metal hydroxide as described by Blight et al. (*J. Chem. Soc.Perkin 1:* 1691 (1974)).

Another DON derivative, 3α-acetoxy-12,13-epoxytrichothec-9-en-8-one-7α,15 carbonate (C₁₈H₂₀O₈, also called "3-acetyl-DON carbonate", designated EN139495 in international PCT Application no. PCT/CA00/00790, filed July 6, 2000) was prepared in 99% yield starting with 20 mg of 3-acetyl-DON, 0.023 ml of pyridine, and 10 mg of triphosgene. The product was obtained as a white solid after silica gel chromatography using 7:3 ethyl acetate-hexane mixture as eluent. Standard nuclear magnetic resonance (NMR) analysis provided the following data indicative the desired molecular structure:
¹H NMR analysis (CDCl₃, 200 MHz): δ: 6.61 (d, J = 8.0 Hz, 1H), 5.36 (m, 1H), 5.29 (s, 1H), 4.49 (d, J = 8.0 Hz, 1H), 4.41 (d, J =16.0 Hz, 1H), 4.19 (d, J = 16.0 Hz, 1H), 3.95 (d, J = 4.0 Hz, 1H), 3.20 (m, 2H), 2.39(s, 1H), 2.12 (s, 3H), 1.92 (m, 1H), 1.92 (s, 3H), 1.12 (s, 3H).

Another DON derivative, 3α-acetoxy-7α,15-benzylidene-12,13-epoxytrichothec-9-en-8-one (C₂₄H₂₆O₈, also called "3-acetyl-DON benzylidene acetal" or "7,15-benzylidene-3-acetyl-DON", designated EN139496, see international PCT Application no. PCT/CA00/00790, filed July 6, 2000) was prepared in 95% yield starting with 20 mg of 3-acetyl-DON and 13 mg of benzaldehyde dimethyl acetal. The product was obtained as a white solid after silica gel chromatography using 4:6 ethyl acetate-hexane mixture as eluent. NMR analysis provided the following data indicative of the desired molecular structure:
¹H-NMR analysis (CDCl₃, 200 MHz): δ: 7.45 (m, 5H), 6.81(d, J = 8.0 Hz, 1 H), 5.39 (s, 1 H), 5.10 (m, 1H), 4.90 (s, 1H), 4.35 (d, J = 8.0 Hz, 1H), 4.31 (d, J = 16.0 Hz, 1H), 3.81 (d, J = 16.0 Hz), 3.81 (d, J = 16 Hz, 1H), 3.21 (m, 2H), 2,20-2.45 (m, 2H), 2.01 (s, 3H), 1.91 (s, 3H), 1.31 (s, 3H).
The ability of a compound to induce fed pattern of gut motility *in vivo* can be determined by the method of Krantis et al. (*Can. J. Physiol., Pharmacol,* 74: 894-903 (1996); see also international PCT application no. PCT/CA00/00790, filed July 6, 2000, incorporated herein by reference).

The effect of a compound described herein on food intake can also be assessed in a feeding study, such as a study using small mammals, such as rodents. In such a study, food consumption and weight gain for each test subject that receives a test compound is assessed and compared to control subjects.

### Example 2. Synthesis of 3α-acetoxy-12,13-epoxy-8α-hydroxytrichothec-9-en-7α, 15-carbonate (EN139499).

The compound of 3-acetoxy-12,13-epoxy-8α-hydroxytrichothec-9-en-7α, 15-carbonate (C₁₈H₂₂O₈, also referred to as "3-acetyl-DON-carbonate-8-ol") was synthesized by the following protocol:

Sodium borohydride (2.4 mg, 0.072 mmol.) was added at 0 °C to a stirred solution of 3-acetyl-DON carbonate (50 mg, 0.13 mmol) in methanol (5 ml). The reduction was complete after 20 minutes as judged by thin-layer chromatography (TLC). The reaction mixture was quenched with 0.1N hydrochloric acid (2 ml), diluted with water (5 ml) and extracted with ethyl acetate (2 x 15 ml). The combined organic layer was washed with sat. sodium bicarbonate solution (10 ml) followed by brine solution (10 ml), and dried over anhydrous magnesium sulfate, and the solvents were removed in vacuo. The crude product was chromatographed (using 1:1 mixture of ethyl acetate:hexane) to yield 50 mg of product (99%) as a white solid.

The structure of the final product, designated EN139499, was confirmed using standard nuclear magnetic resonance (NMR) analysis, which yielded the following data:
¹H NMR [CDCl₃, 200 MHz]: δ: 5.98 (d, J = 8.0 Hz, 1H), 5.45 (d, J *=* 16 Hz, 1H), 5.18 (m,1H), 5.02 (d, J = 16 Hz, 1H), 4.78 (d, J = 8.0 Hz, 1H), 3.72 -3.88 (m, 2H), 3.12 (m, 2H); 2.60 2.72 (m, 1H), 2.12 (s, 3H), 2.02 - 2.18 (m, 1H), 1.92 (s, 3H), 1.01 (s, 3H).
The above synthesis and analysis indicated that EN139499 has the structure shown in Figure 1A.

A single dose of the carbonate compound EN139499 (10 mg·kg⁻¹) was tested for ability to induce the fed pattern of gut motility in rats using the method of Krantis et al. (1996). Within 30 seconds of intravenous injection of EN139499, there developed a long lasting (40 min, n = 10) hyperactivity in the duodenal sites (D1, D2) and a simultaneous and parallel attenuation of motor activity in the gastric antrum (S1). Examples of these effects on *in vivo* gastrointestinal action are presented in the recording of gut motility in Figure 2.

EN139499 administered intravenously to test subjects at a dose of 10 mg-kg⁻¹ body weight (b.w.) induced the fed pattern of gut motor activity. Bar graphs in Figures 3-14 show the effect of EN139499 (open bars) on the amplitude and frequency of relaxations and contractions of the gut at duodenal sites D1 and D2 and gastric antral site S1 expressed as a percent of the activity of the control "group" activity (diagonal bars) of the fasting pattern, which is taken as 100 percent. In particular, EN139499 stimulated gut motility in duodenal D1 relaxation amplitude and frequency as shown in Figures 3 and 4, respectively, and in duodenal D1 contraction amplitude and frequency (Figures 5 and 6, respectively). A similar effect was found at the D2 duodenal site (see, Figures 7-10). For comparison, Figures 3-10 also show the relatively quiescent "intergroup" activity (filled bars). EN139499 also produced a pronounced decrease in gastric antral (S1) relaxation amplitude and contraction (Figures 11 and 12, respectively) and contraction amplitude and contraction (Figures 13 and 14, respectively). The stimulation of duodenal gut motor activity accompanied by the contemporaneous decrease in gastric antral motor activity indicate that administration of EN139499 to the test subjects clearly stimulated the'fed pattern of gut motility.

Other data indicated that EN139499 at 1 mg·kg⁻¹ could also induce a similar level of fed pattern motility, however the duration of action was 20-30 minutes.

### Example 3. Synthesis of 3α,8α-diacetoxy-12,13-epoxytrichothec-9-en-7α,15-carbonate (EN139500).

The compound 3α,8α-diacetoxy-12,13-epoxytrichothec-9-en-7α,15-carbonate (C₂₀H₂₄O₉, also referred to as "3,8-diacetyal-DON carbonate") was synthesized using the following protocol:

Acetic anhydride (1.32 mmol) was added to a at 0 °C solution of the 8-hydroxy-3-acetyl-DON carbonate (EN 139499, as synthesized above) (25 mg, 0.066 mmol), triethylamine (1.32 mmol) and DMAP (1 mg) in anhydrous CH2Cl2 (4ml), acetic anhydride (1.32 mmol). The solvent was evaporated in vacuo after 6 hours. The crude reaction product was purified by column chromatography using the 1:1 mixture of ethylacetate:hexane to yield 23 mg (95%) of final product.

The structure of the final product, designated EN139500, was confirmed using standard NMR analysis, which yielded the following data:
¹H NMR (CDCl₃): δ: 5.86 (d, J = 8.0 Hz, 1H), 5.48 (d, J =16 Hz, 1H), 5.18 (m, 1H), 5.08 (d, J =16 Hz, 1H), 4.55 (d, J = 8.0 Hz, 1H), 4.44 (d, 1H), 4.10 (d, 1H), 3.78 (d, 1H), 3.14 (m, 2H), 2.18 - 2.26 (m, 2H),2.12 (s, 3H),1.99 (s, 3H), 1.93(s, 3H), 1.03 (s, 3H).
The above synthesis and NMR analysis indicated that EN139500 has the structure shown in Figure 1A.

### Example 4. Synthesis of 3-acetoxy-7α,15-benzylidene-12,13-epoxy-9,10-dihydrotrichothecin-8α-ol (EN139501).

The compound 3α-acetoxy-7α,15-benzylidine-12,13-epoxy-9α-methyltricohthecan-8α-ol (C₂₄H₃₀O₇) was synthesized using the following protocol:

Sodium borohydride (3.6 mg, 0.11 mmol) was added to a stirred solution of 7,15-benzylidene-3-acetyl-DON (50 mg, 0.13 mmol, EN139496 described above) in 3 ml of methanol at 0 °C. The reaction mixture was quenched after 30 m with 0.1N hydrochloric acid (5 ml) and extracted with ethyl acetate (2 × 15 ml). The combined organic layer was washed with brine solution (15 ml), dried over anhydrous magnesium sulfate and evaporated in vacuo. Purification via column chromatography (3:7 mixture of ethylacetate:hexane) afforded 50 mg (>99% yield) of EN139501 as white solid.

A preferred method of synthesizing EN139501 was by reduction of 3-α-acetoxy-7α,15-benzylidene-12,13-epoxytrichothecan-8-one (designated Compound EN139519; see, Example 7) with NaBH₄ in methanoL The reduction of EN139519 was carried out using the same protocol described for the reduction of EN139496 to EN139519 (see, Example 7). The product, a white solid, was obtained in greateer than 90% yield.

The structure of the final product was confirmed by standard NMR and mass spectroscopy (MS) analyses, which yielded the following data:
¹H and ¹³C NMR analysis:
1H NMR(acetone-d₆, 500 MHz) δ: 0.97 (d, J = 6.8Hz., 3 H), 1.26 (s, 3H), 1.93-1.99 (td, 1H), 1.95 (s, 3H), 2.00-2.14 (m, 1H) , 2.29 (dd, J = 15,4.4 Hz, 1H), 3.04 (d, J = 4.0 Hz, 1H), 3.10 (d, J = 4.0 Hz, 1H), 3.66 (d, J =4.5 Hz, 1H), 3.78 (t, J = 2.0 Hz, 1H), 4.04(d, J = 7.2 Hz, 1H), 4.18(d, J =11.8 Hz, 1H), 4.30 (m 2H), 4.49 (d, J = 5.0 Hz , 1H), 5.02(td, J = 11.2,4.4 Hz, 1H), 7.02 (s, 1H), 7,25-7.33(m, 3H), 7.43 (-74.6 (m, 2H).
¹³C NMR (acetone-d6, 125,MHz) d: 14.5, 17.6. 18.1, 20.8, 20.8. 30.78, 40.0. 47.3, 48.5. 65.9, 67.6, 72.3, 72.6, 75.0, 77.2, 78.6, 97.0, 127.2, 128.6, 128.8, 141.1, 170.7,
Mass Spectrum: 430 M+, 37), 429 (25), 373 (4.3) 105 (100).

The above data indicate the following structure for EN139501:

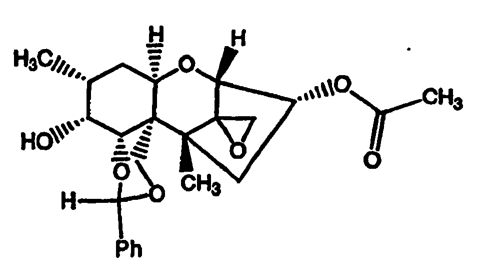

Compound EN139501 was at times obtained directly by reduction of EN139496 without the isolation of the intermediate EN139519.

### Example 5. Synthesis of 7α,15-benzylidene-9α,12β-dimethyltrichothecan-3α,8α,12α-triol (EN139502).

The compound of 7α,15-benzylidene-9α,12β-methyltrichothecan-3α,8α,12α-triol (C₂₂H₃₀O₆) was synthesized using the following protocol:

Lithium aluminium hydride (3.7 mg, 10 mmol) was added to a solution of EN 139501 (25 mg, 0.067 mmol., as synthesized above) in dry tetrahydrofuran (5 ml) stirred at 0 °C. After 15 min of reaction time, the ice bath was removed and the reaction mixture was refluxed for 1 hour. Then the reaction mixture was cooled to 0 °C and quenched by the dropwise addition of the cold water. The solvent was evaporated in vacuo and the residue was extracted with the dichloromethane (3 x 15 ml). The combined organic layer was washed with the brine solution (15 ml), dried over magnesium sulfate and the solvents were evaporated in vacuo. Purification by column chromatography (using 7:3 mixture of ethylacetate:hexane) gave 18 mg of the product EN139502 as a white solid (72% yield).

The structure of the final product, designated EN139502, was confirmed by standard mass spectroscopy and NMR analysis, which yielded the following data:
Mass spectroscopy: MS (EI): 390.(M+)
NMR analysis:
³H NMR (acetone-*d*₆,500 MHz): δ: 7.28-7.58 (m, 5H), 6.85 (s, 1H), 4.58 (m. 3H), 4.42 (d, 1H), 4.28 (m, 1H), 4.18 (m, 1H), 4.04 (m, 1H), 3.92 (m, 1H), 3.52 (d, 1H), 2.20-2.18 (m, 3H), 1.52 (s, 3H), 1.48 (s, 3H), 0.98 (d, 3H).
¹³C NMR (acetone-*d*₆): 141-49, 128.72, 128.51, 127.35, 97.43, 83.13, 83.11, 80.25, 77.36, 75.26, 71.29, 70.52, 69.83, 53.02, 43.33, 41.53, 31.42, 30.62, 22.57, 18.47, 17.92, 14.44
The above synthesis and NMR analyses indicated that EN139502 has the structure shown in Figure 1B. EN139502 lacks the 9,10 double bond, the 8-keto, and the 12,13-epoxide characteristic of naturally occurring trichothecenes. EN139502 did not induce the fed pattern gut motility as measured by the method of Krantis et al. (*Can. J. Physiol*. *Pharmacol., 74:* 894-903 (1996), incorporated herein by reference). However, EN139502 did appear to intensify or enhance the fasting pattern after a relatively short delay (approximately 5 min). The resulting fasting pattern was robust and without any change in the period or time course of the pattern with respect to the intergroup period and the length of the MMC (i.e., the group activity). In particular, the intergroup and group activities became pronounced. This was most obvious in animals where the control recording of the fasting pattern was weak.

These data indicate that certain compounds like EN139502 may be used to increasing eating or feeding time, and, thereby, increase food intake and weight gain, or to counteract or modulate the effect of other compounds described herein that induce the fed pattern.

The data for EN139502 and those for compounds EN139499, EN139500, and EN139501 also provide new insight into the the importance of structural features previously thought to be essential for the ability of trichothecenes to reduce food intake or induce feed refusal. Traditionally, the characterisitic 9,10 double bond and the 8-keto group of natural occurring trichothecenes have been considered essential for such activity. However, the data provided herein indicate, surprisingly, that neither of these groups appears to be necessary for inducing fed pattern of gut motor activity and, therefore, reducing.food intake. In particular, EN139499 contains a 9,10 double bond and 12,13-epoxy group characterisitic of trichothecenes, but the 8-keto group has been reduced to an alcohol. Nevertheless, EN139499 induced the fed pattern of gut motility. Likewise, EN139500 also induced fed pattern even though this compound possesses an 8-acetoxy group instead an 8-keto group. These data indicate that the characteristic 8-keto group of trichothecenes is not necessary for inducing fed pattern of gut motility. In addition, reduction of the 9,10 double bond as well as the 8-keto group, as in EN139501 did not eliminate the ability to induce the fed pattern of gut motility. Thus, contrary to the view of the prior art, the 9,10 double bond is also not essential for the ability to induce fed pattern of gut motility.

However, EN139499, EN139500, and EN139501 retain the 12,13-epoxide ring. This 12,13-epoxide appears to be important for inducing fed pattern and reducing food uptake, as illustrated by EN139502, in which not only have the 9,10 double bond and the 8-keto group of classic trichothecenes been reduced, but the 12,13-epoxide ring has been opened as well. As noted above, unlike EN139499, EN139500, and EN139501, the compound EN139502 did not induce the fed pattern of gut motor activity.

### Example 6. Synthesis of 3α-acetoxy-12,13-epoxy-9α-methyltrichothecan-7α,8α,15-triol (EN139503).

The compound designated EN139503 (C₁₇H₂₆O₇) was synthesized using the following protocol:

A solution of EN 139501 (25 mg, 0.067 mmol) and camphor sulfonic acid (23.31 mg, 0.1 mmol) dissolved in 5 ml of methanol was stirred for 90 min at room temperature. The reaction mixture was diluted with 20 ml of ethyl acetate, washed with water, saturated sodium bicarbonate solution and brine solution. The organic layer was dried over magnesium sulfate and the solvents were evaporated in vacuo. The crude product was chromatographed using 6:4 mixture of ethylacetate:hexane to yield 19.3 mg (>99%) of the product, EN139503, as white solid.

The structure of the final product, designated EN139503, was confirmed by NMR analysis, which yielded the following data:
¹H NMR(CDCl₃, 500 MHz): δ: 5.12 (m, 1H), 4.28 (d, 1H), 3.93 (m, 2H), 3.80 (m, 1H), 3.75 (d, 1H), 3.66 (d, 2H), 3.12 (m, 2H), 2.04-2.10 (m, 2H), 2.05 (s, 3H), 1.76-1.84 (m, 1H), 1.50 (dt, 1H), 1.22-1.25 (m, 1H), 1.08 (s, 3H), 0.97 (d, 3H).
¹³C NMR(CDCl₃, 500 MHz): δ: 170.54, 78.28, 76.30, 73.06, 72.62, 71.40, 64.83, 63.16, 48.10, 47.99, 46.83, 41.03, 30-81, 29.43,20.84, 17.05, 14.87
The above synthesis and NMR analyses indicated that EN139503 has the structure shown in Figure 1B.

### Example 7. Synthesis of additional compounds.

As outlined below, additional new compounds were readily synthesized from various "starting" or "parent" compounds using organic synthesis procedures known in the art.

Debenzylidenations were carried out by dissolving the starting compound in methanol, adding a catalytic amount of camphorsulfonic acid, and stirring at room temperature until completion of reaction as monitored by thin-layer chromatography (TLC). The usual reaction time was 1 hour.

Carbonylations were accomplished by reaction of the 7α,15-diol with triphosgene and pyridine in dichloromethane at room temperature. The usual reaction time was 6 hours.

Acetylations and benzoylations were performed with acetic anhydride and benzoyl chloride, respectively, in dichloromethane at room temperature. Triethylamine and 4-dimethylaminopyridine (DMAP) were used as co-reagent and catalyst, respectively.

9α,12β-dimethyltrichothecan-3α,7α,8α,12α,15-pentaol (designated EN139504, Figure 1B), was synthesized from EN139502 by debenzylidenation.

3α-acetoxy-12,13-epoxy-9α-methyltrichothecan-7,8,15-triol (designated EN139503, Figure 1B) was synthesized by debenzylidenation of EN139501.

7α,15-benzylidene-3α,8α-diacetoxy-12,13-epoxy-epoxy-9α-methyltrichothecane (designated EN139505, Figure 1C) was synthesized by acetylation of EN139501.

3α,8α-diacetoxy-12,13-epoxy-9α-methyltrichothecan-7α,15-diol (designated EN139506, Figure 1C) was synthesized by debenzylidenation of EN139505.

3α,8α-diacetoxy-12,13-epoxy-9α-methyltrichothecan-7α,15-carbonate (designated EN139507, Figure 1C) was synthesized by carbonylation of EN139506.

7α,15-benzylidene-3α,8α-diacetoxy-9α,12β-dimethyltrichothecan-12α-ol (designated EN139508, Figure 1D) was synthesized by acetylation of EN139502.

3α-acetoxy-12,13-epoxy-9α-methyltrichothecan-8-one-7α,15-carbonate (designated EN139511, Figure 1E) was synthesized by hydrogenation of EN139495 (3α-acetoxy-12,13-epoxytrichothec-9-en-8-one-7α,15-carbonate; described above).

3α-benzoyloxy-12,13-epoxytrichothec-9-en-8-one-7α,15-carbonate (designated EN139514, Figure 1E) was synthesized by benzoylation of EN139494.

EN139495 was hydrogenated with H₂Pd/C to afford EN139512.

12,13-Epoxytrichothec-9-ene-3α,7α,8α,15-tetraol (designated EN139518) was synthesized by saponification of EN139495 in aqueous methanolic KOH. The structure of EN139518 is given below:

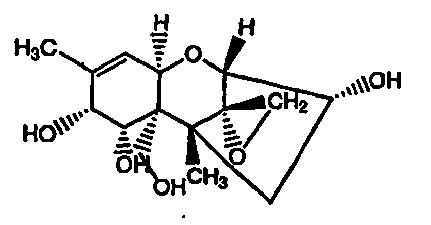

Synthesis of 3-α-acetoxy-7α,15-benzylidene-12,13-epoxytricothecan-8-one (designated EN139519) by reduction of EN139496 with NaBH₄ in methanol:

Sodium borohydride (65 mg) was added to a solution of 426 mg of EN139496 in 40 ml of methanol at 0°C. The reaction mixture was then allowed to warm to room temperature and stirred until TLC showed the disappearance of the staring material, typically 1 hour. Then 25 ml of a 1% HCl solution was added and most of the methanol was removed under reduced pressure. The remaining aqueous solution was extracted 3 times with 20 ml of ethyl acetate. The combined organic extracts were dried over sodium sulfate and the solvent was removed. The purified product 410 mg (>95%) was obtained after silica gel chromatography using 2:3 ethyl acetate-hexane as eluent. The structure of EN139519 is shown below: 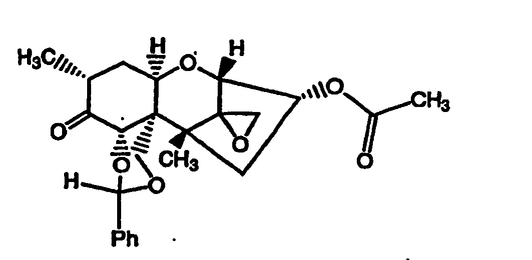 ¹H NMR (CDCl₃, 200MHz) δ: 1.1(d, 3H) 1.3 (s,3H) 1.7-1.9(m,1H), 2.03 (s, 3H), 2.0-2.28 (m, 3H), 2.6-3.05 (m, 1H), 3.05 (d,1H), 3.1 (d, 1H), 3.6(d,1H), 3.8-3.9(m, 2H), 4.15 (d, 1H), 4.85 (s,1H), 5.25 (td, 1H), 5.7 (s, 1H), 7.25-7.7 (m, 3H), 7. 45-7.55 (m, 2H).
¹³C NMR δ: 12.5, 16.4, 20.2, 35.5, 36.8, 39.8, 46.8, 47.6, 47.9, 65, 67.5, 69.8, 70.7, 78, 79.5, 97.6, 125.9, 128, 128.5, 137, 170.3, 208.

### Synthesis of 7α,15-benzylidene-12,13-epoxy-3β-hydroxytrichothecan-8-one (designated EN139520) and 7α,15-benzylidene-9α,12β-dimethyl-3α,12α-dihydroxytrichocethcan-8-one (designated EN139522):

A suspension of 135 mg of LiAlH₄ in 5 ml of THF was cooled to 0°C and a solution of 757 mg of Compound EN139496 in 25 ml of THF was added drop-wise over 30 minutes. The solution was allowed to warm to room temperature and then heated to reflux for 1 to 2 hours. The mixture was cooled again to 0°C and quenched by addition of ice water. Most of the THF was removed under vacuum and the remaining solution was acidified to pH 5 and extracted 3 times with 40 ml of ethyl acetate. The combined organic extracts were dried with Na₂SO₄ and the solvents were evaporated. The crude product was chromatographed on silica geL Elution with 2:3 ethyl acetate afforded 150 mg of EN 399520. Elution with 4:1 ethyl acetate gave 168 mg EN 399522.

The structural data for 7α,15-benzylidine-12,13-epoxy-3β-hydroxytrichothecan-8-one (Compound EN139520) is given below: 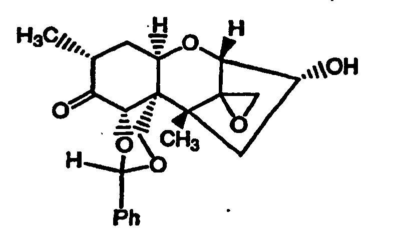 ¹H NMR (CDCl₃, 500 MHz) δ: 1.11 (d, J = 6.5Hz, 3H), 1.30 (s, 3H), 1.78 (td, J = 14.3, 2.6 Hz, 1H) 2.03 (dd, J = 14.1, 4.1Hz, 1H), 2.07 (dd, J = 14.1, 4.1 Hz, 1H), 2.08 (bs, OH), 2.15-2.2 (m, 2H), 2.95-3.07 (m, 1H), 3.07 (d, J = 4.1Hz, 1H), 3.11 (d, J = 4.1Hz, 1H), 3.59 (d, J = 4.5 Hz, 1 H), 3.61 (d, J =12.6 Hz, 1H), 4.17 (dd, J = 12.3, 1.9 Hz, 1H), 4.56 (td, J = 10.7, 4.4 Hz, 1H), 4.88 (s, 1H), 5.72(s, 1H), 7.3-7.38 (m, 3H), 7.43-7.5.(m. 2H).
¹³C δ: 13.4, 16.9, 35.6, 37.5, 43.0, 47.6, 47.8, 48.4, 65.7, 67.7, 69.4, 70.2, 79.7, 80.2, 97.5, 126.3, 128.3, 129.0, 137.6,206.6.

The structural data for 7α,15-benzylidene- 9α,12β-dimethyl-3α,12α-dihydroxytrichocethcan-8-one (Compound EN139522) is given below: 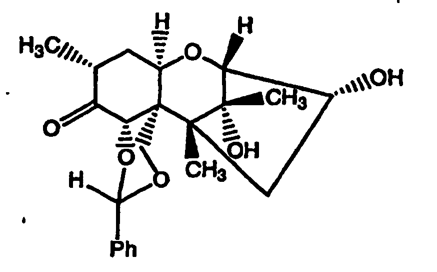 ¹H NMR (acetone-d₆, 500 MHz) δ: 1.02 (d, J = 7.0 Hz, 3H), 1.41 (s, 3H), 1.59 (s, 3H), 1.76 (dd,J= 14.1,3.2 Hz, 1H), 1.88 (td, J = 14.2, 3.3 Hz, 1H), 2.17 (dd, J= 14.1, 10.5 Hz, 1H), 2.88 (bs, 1H), 2.96 (m, 1H), 3.49 (d, J = 12.7 Hz, 1H), 3.60 (d, J = 4.6 Hz, 1H), 3.82 (s, 1H), 4.0-4.08 (m, 1H), 4.12 (dd, J = 12.6, 1.4 Hz, 1H), 4.16 (t, J = 1.4Hz, 1H), 4.64-4.68 (m, 1H), 5.08 (s,1H) 5.78 (s, 1H), 7.3-7.4 (m, 3H), 7.5 (m, 2H).
¹³C NMR δ: 14.1,17.3, 22.4, 37.4, 38.9, 43.9, 50.2, 52.8, 69.2, 70.5, 71.3, 77.1, 80.1, 80.2, 97.0, 127.2, 128.9, 128.3, 139.9, 209.2.

### Example 8. Feeding studies in rats.

A protocol for a feeding study using adult Sprague-Dawley rats was adopted from the protocol for a trichothecene feeding study using pigs described by Prelusky (*Natural Toxins, 5(3):* 121-125 (1997)). From *in vivo* motility screening studies it was determined that the trichothecene DON at 1-10 mg/kg, body weight (b.w.), intravenous (iv) induced the fed pattern of gut motility in adult Sprague-Dawley rats. Given a 300 gram (g) rat, this is equivalent to 0.3-3.0 milligrams (mg) of DON actually injected. The dietary concentration range (ppm) of compounds tested in a DON feeding study were chosen to match the intravenous bolus dose given in *in vivo* motility studies, as well as, to be in accordance with other feeding/toxicology studies in the literature. Using data provided by Arnold et al. (*Fundament. Appl. Toxicol., 6(4):* 891-696 (1986)), stating that 0.25 mg/kg b.w. of DON corresponded initially to 1.69 ppm, the amount of DON ingested by a rat eating an average of 22 g food/day (a normal daily food intake for an adult rat) is 0.037mg. In the study by Prelusky (1997), pigs received approximately 1.26 mg/kg (of body weight) of DON/day, which by extrapolation is equivalent to 0.378 mg DON/day for a 300 g rat.

Newly synthesised compounds that induced fed pattern in the gut motility assay of Krantis et al. (1996) were further evaluated for their effect on food consumption and weight gain in rats. In these feeding studies, three dietary concentrations were chosen for both the test compound and the generic DON trichothecene (positive control), along with 'no-drug' control groups. As indicated below, another group acted as a negative control. Another control group (pair-fed group), as described below, helped to unconfound data due to changes in food consumption versus drug effects.

The dietary test concentrations, based upon an animal body weight (b.w.) of 300 g, were: 0.083mg/day (3 ppm); 0.276 mg/day (10 ppm); and 0.55 mg/day (20ppm). From the results of a pilot feeding study to measure the daily consumption of normal feed by male Sprague-Dawley rats, the rate of daily consumption was an average of 27.6 g/day. When bioavailability was considered (Arnold et al., 1986), the daily uptake for DON or a test compound was calculated to be 0.28 mg/kg b.w./day, 0.92mg/kg bw/day, and 1.38 mg/kg b.w./day; respectively for each dietary test concentration.

For each feeding experiment, the following protocol was used. Twenty-five male Sprague-Dawley rats weighing between 175-200 g obtained from Charles River, Quebec Canada, are allowed to acclimatize for 6 days. Rats are randomly distributed into groups of ten (n =10) for study:
Group 1 was a control group, which receives unaltered food and water ad libitum throughout the study;
Group 2 was a "Pair-Fed" control group, which is begun two days behind (8 days to acclimatize) and receive food limited to the average amount eaten by test animals two days prior (n = 5 paired to group 4, n=5 paired to group 5);
Group 3 is a DON-fed group (when included in study), which received food containing 3,10, or 20 ppm of deoxynivalenol (DON), and water ad libitum;
Group 4, Group 5, etc. are experimental groups fed a test compound described herein (such as EN139499 and EN139500), which receive food containing 3,10, or 20 ppm test compound, and water ad libitum; respectively.

The feeding trial is begun with all groups receiving 3 days of normal (control) food (day -3 to -1), during which time baseline variations in food consumption and weight gain are monitored. For the next seven, or ten days (day 1 to 10), each rat receives their designated experimental dietary schedule. Animals are weighed daily. Fresh food is provided daily and leftover food (as well as dry, spilled food recovered from beneath the feed crucible) is also weighed daily. The amount of food provided throughout the day is recorded.

All animals are fed a standard rat feed, which is provided in powdered form (18% Autoclavable Rodent Feed, Purina Mills, Woodstock, ON). For each treatment group, test compound (in powdered form) is thoroughly mixed in with the food to provide the required final concentration.

Each rat is kept in its own cage. Two floor racks are positioned in the bottom of each cage on top of the wood chips. This ensures animals only eat the food provided. Powdered food is placed in specially manufactured porcelain crucibles (designed to limit food spillage) within the cage. The animals are maintained on a 12 hour light/dark cycle, and entrance to the experimental room is restricted to minimize triggering any unnatural eating behavior due to any extraneous activity and/or noise. Cages are changed, animals weighed, old food weighed and new food provided at the beginning of each day's light period (approximately 9:00 a.m.). This routine is carried out carefully to minimize any stress on the animals. Each rat has one plastic tube positioned in the cage in which to hide.

Over each of up to 13 days (day -3 to 10), animals are weighed and food consumption determined. Either day 7 or day 10 marks the completion of the experiment. Two animals from each group are euthanized, and samples of the stomach, small and large intestines are dissected out and processed for histopathological examination.

During Days -3 to -1, baseline variations in food consumption and weight gain for the designated dietary treatment groups have not been found to significantly differ from the control group.

For all DON and test compound dietary treatment groups, there was a reduction in food consumption and weight gain, evident from day 1 of the treatment diet.

When EN139499 and EN139500 (20 ppm) were tested in this protocol, both food consumption and weight gain were reduced in animals receiving this compound in their diet. The effect of EN139499 on daily weight gain was followed over 10 days (Figure 15). For comparison purposes, the daily profile for both the EN139495 (3-acetyl-DON) treatment group and the EN139499 pair fed group are shown in Figure 15. Overall, the weight gain for the EN139499 group at day 10, was reduced by 19%. Similar results were obtained for the EN139500 group.

The results of such feeding studies showed that DON (positive control) and EN139499 (test compound), administered through the diet, caused an immediate and sustained reduction in food consumption and in weight gain in Sprague-Dawley rats. Each of these compounds was chosen for testing based upon an evaluation of their capacity to induce fed pattern of gut motility using the method of Krantis et al. (1996), described above. Compounds active in the gut motility assay of Krantis et al. (1996) displayed a similar profile of action as DON, i.e., within 20-120 seconds of injection in anesthetized Sprague-Dawley rats, the spontaneous fasting motor pattern of the gastroduodenum changed to a typical fed pattern motor activity. This effect lasted 30-60 minutes and then the spontaneous motor activity recovered to a fasting pattern. According to the invention, a compound that induces this fed pattern artificially shortens feeding time and, hence, reduces overall food intake in sensitive species.

The results of the feeding studies on representative compounds of the invention demonstrated this effect. In particular, DON and test compounds described herein produced their most dramatic food consumption on the first day, and the effect did not recover (data not shown). The result of decreased food consumption was a detectable decrease in weight gain.

In conclusion, the ability of this feeding protocol to readily detect daily changes in feeding behavior has shown a consistent and similar profile in the action of DON (positive control) and the test compounds. Moreover, the results show the efficacy of the *in vivo* motility assay of Krantis et al. (1996) to screen for compounds that can regulate food intake. The feeding study results indicated that DON, EN139495, and test compounds of the invention, EN139499 and EN139500, when given in the diet at concentrations equivalent to or below the maximum concentration of DON tested in rats in other studies, show a concentration-dependant reduction in food consumption and weight gain. The effectiveness of these new compounds to each other was similar. In these studies, the effect of the test compounds against the effects of equivalent dietary concentrations of DON were compared and shown to have a similar profile of action and comparable potency.

### Example 9: Additional study of EN139499-dependent induction of fed pattern.

The previous study of compound EN139499 (Example 2, above) showed that when administered systemically (i.e., 10 mg/kg, iv) to Sprague-Dawley rats, the compound induced a classic fed pattern motor activity in the rat gastroduodenum as measured by the method of Krantis et al. (1966). The study of EN139499 was extended to include an evaluation of the compound at the lower dose of 1 mg/kg (iv) (n=6).

EN139499 was prepared in 0.9% saline for administration to halothane-anesthetized rats, and gut motor activity recording using the method of Krantis et al. (1996) as described above. The results of this study clearly showed that EN139499 was also effective at inducing the classic fed pattern of motor activity in rats when administered at the lower dose of 1 mg/kg (iv). Specifically, in the small intestine duodenum (site D1), EN139499 induced both increased amplitude and frequency for both contractions and relaxations. Consistent with induction of the classic fed pattern of gut motor activity, the stomach (gastric antrum site S1) displayed a typical and significant reduction of motor activity (data not shown).

When compared to the earlier result using the compound at 10 mg/kg (iv), a clear dose response was evident: the time of onset of action for EN139499 at the low dose was 120 seconds from injection compared to 70 seconds for the higher dose and the duration of action for the lower dose was 20-30 minutes compared to 40 minutes for the higher dose.

### Example 10: Induction of fed pattern of gut motility by other compounds.

A number of other compounds were further tested for the ability to induce fed pattern of gut motility in rats as determined by the gut motility assay of Krantis et al. (1996). Induction of a classic fed pattern of gut motility was evident from the characteristic recording patterns in the stomach (e.g., gastric antrum site S1) and small intestine (duodenum site D1 and/or D2) and from the analysis of individual components of the recorded motor activity (i.e., amplitude and frequency of contractions and relaxations) as shown above for compound EN139499 (see, e.g. Figs. 7-14). Particular results of an analysis of data for each compound are summarized briefly below.

EN139506 and EN139507 were dissolved in 20% dimethyl sulfoxide (DMSO). EN139503 was dissolved in 40% DMSO. EN139505 and EN139508 were dissolved in 70% DMSO. EN139518 was dissolved in 0.9% saline. DMSO has been tested in the *in vivo* protocol described herein at up to a 70% solution and found to have no effect on control gut motor activity recorded from the gastroduodenum (sites S1, D1, D2) as determined using the method of Krantis et al. (1996).

Under control conditions, the gastric antrum of halothane anesthetized male Sprague-Dawley rats displayed spontaneous, small amplitude oscillatory contractile and relaxant motor response. In the proximal duodenum (site D1) and lower down in the small intestine at the level of the proximal ileum (site D2), sponstaneous motor activity was patterned into recurring cycles of periods of intense propagating motor activity (MMC or group activity) and non-propagating motor activity (intergroup activity) with a cycle length of 6-9 minutes. The MMC or group activity ranged from 1 - 4.5 minute duration and copmrised high amplitude, high frequency relaxations and contractions. The interposed, non-propagating motor activity comprised primarily of low amplitude, low frequency relaxations and contractions. Thus, all control patterns of motor activity were consistent with all previous studies (see, above).

At 10 mg/kg (of body weight), iv (n=10), the compound EN139503 induced classic fed pattern in rats as determined by the gut motility assay of Krantis et al. (1996) with a duration of action of 40-50 minutes and an onset time of 60 seconds after administration.

An example of the effect of EN139503 on the small intestine (site D1) is shown in Fig. 16. Administration of EN139503 (vertical arrow) was followed (horizontal arrow) by a classic fed pattern of gut motor activity as shown in the duodenum recording (site D1) as an induction of an intense pattern of hyperactivity. Consistent with authentic fed pattern, there was a simultaneous decrease in recorded tissue motor activity in the gastric antrum (site S1) (date not shown). As expected, an analysis of the individual components (amplitude and frequency) of gut motor activity also indicated induction of a classic fed pattern of gut motor activity occurred in the small intestine and stomach (data not shown).

The compound EN139506 was tested at 10 mg/kg, iv (n=8), and found to induce a classic fed pattern of gut motor activity similar to that of EN139503. The duration of action of EN139506 was 30-40 minutes with an onset time of 120 seconds after administration.

The compound EN139507 was tested at 10 mg/kg, iv (n=8), and was found to induce a classic fed pattern of gut motor activity. The time of onset of induction of fed pattern by EN139507, i.e., 120 seconds, was similar to that seen with a number of compounds tested, although the time of duration of the fed pattern, i.e., 15 minutes, was somewhat shorter in duration than the 30-40 minute range recorded for a number of compounds, such as EN139503 and EN139506.

The compound EN139505 was tested at 20 mg/kg, iv (n=5). A fasting pattern of gut motor activity was occasionally observed which lasted 30-35 minutes. Onset time for activity at duodenal site D1 was 60 seconds. In the stomach, a reduction in motor activity, typical of the fed pattern, was recorded with an average onset time of 84 seconds after injection (data not shown).

Compound EN139508 induced a dose-dependent fed pattern of gut motility when tested at 10 and 20 mg/kg, iv. The higher dose displayed a longer lasting action (approximately 30 minutes). At both doses, onset of action was approximately 60 seconds after administration.

The compound EN139518 was tested at 10 mg/kg, iv (n-8), and found to induce a fed pattern of gut motor activity within 120 seconds of administration. The compound had a duration of action of 20-34 minutes.

Compound EN139519 was tested at a dose of 10 mg/kg, iv, (n=7), and found to induce a fed pattern with a time to onset of 25 seconds and duration of 30-50 minutes.

Compound EN139522 was tested at a dose of 10 mg/kg, iv, (n = 6), and found to induce a classic fed pattern with a time of onset of 20 seconds and duration of 35 minutes.

### Example 11. Additional feeding studies in rats.

Additional studies were carried out to study the effect of selected compounds of the invention on feeding and weight gain using basically the same method as described above in Example 8. Unless otherwise indicated, these studies consisted of a 13-day protocol in which weight gain and food consumption were monitored in male Sprague-Dawley rats that received food containing a selected compound of the invention. Rats were fed standard rat feed in powdered form (Purina Mills, Woodstock, ON) with or without test compound.

In these studies, male Sprague-Dawley rats weighing between 175-200 g (Charles River, Quebec, Canada) were allowed to acclimatize for 6 days. Rats were randomly distributed into the following five groups for study:
Group 1 (control) received unaltered food and water *ad libitum.*
Group 2 received feed containing 3, 10, or 20 ppm of DON and water *ad libitum.*
Groups 3 and 4 (test groups) received food containing a selected compound of the invention.
Group 5 (pair-fed), when included in study, rats in this group were two days behind (8 days to acclimatize) and received food limited to the average amount eaten by test animals (Group 3 or 4) two days prior.

The feeding trial began with all groups receiving three days of normal (control) food (day -3 to -1), during which time baseline variations in food consumption and weight gain were monitored. For up to ten days (day 1 to 10), each rat received their designated experimental dietary schedule of fresh food alone or fresh food and test compound. Animals were weighed daily. Fresh food was provided daily. Leftover food as well as dry, spilled food was recovered from beneath the feed crucibles and weighed daily. The amount of food provided throughout the day was recorded.

Each rat was kept in its own cage, which was designed to insure the animals only ate the food provided. Powdered food was placed in specially manufactured porcelain crucibles designed to limit food spillage. The animals were maintained on a 12 hour light/dark cycle, and entrance to the experimental room was restricted to minimize triggering any unnatural eating behavior due to any extraneous activty and/or noise. Cages were changed, animals weighed, old food weighed, and new food provided at 9:00 AM each day. This routine was carried out carefully and at the same time each day. Each rat had one plastic tube positioned in the cage in which to hide.

Over each of up to 13 days (i.e., day -3 to 10), animals were weighed and food consumption determined. An experiment was completed on day 5, 7, or 10. Two animals from each group were euthanized, and segments of the stomach, small intestine, and large intestine were dissected out and processed for histo-pathological examination.

The above protocol was used to study and compare the effect of DON and other compounds on food intake and weight gain.

### Study 1

In Study 1, rats were fed normal powdered feed (normal diet) mixed with EN139499 at 20 ppm (n = 8) or at 40 ppm (n = 5); EN139500 at 20 ppm (n = 8) or at 40 ppm (n = 6); DON at 20 ppm (n = 6); or no compound (control, n = 6).

Rats fed normal diet mixed with DON (20 ppm) showed a decrease in both daily consumption of feed and average daily weight gain compared to the control group. Daily consumption of food by rats fed normal diet mixed with EN139499 or with EN139500 exhibited a "see-saw" pattern compared to control animals, which is also a typical pattern for animals receiving feed mixed with DON (data not shown).

Overall weight gain at day 10 of rats fed an EN139499 diet at 40 ppm was 63.6% of the control group (normal diet alone) (see, Figure 16). This result in the observed overall weight gain in rats fed an EN139499 at 40 ppm diet was approximately twice the effect observed in rats fed an EN139499 at 20 ppm diet. By contrast, EN139500 was equally effective as EN139499 at 20 ppm and no longer effective at 40 ppm, indicating that the concentration response range for these two compounds is different.

### Study 2

In Study 2, rats were fed normal diet mixed with EN139518 at 20 ppm (n = 10) or no compound (normal diet alone control, n = 6). EN139518 displayed excellent consistency as a powder and was fully soluble. Such properties are particularly beneficial for incorporating the compound into food product, such as the normal powdered feed used in this study.

A typical "see-saw" pattern was observed in the percent change in daily weight gain (data not shown). "Normalized" daily consumption by rats fed the EN139518 diet reached and maintained significant decreases compared to control group rats fed normal diet alone throughout Day 3 to Day 5 of the study (see, Figure 17). Rats fed the EN139518 diet showed an approximately 16% reduction in weight gain by Day 5 of the study compared to control group rats (Figure 18).

### Study 3

In Study 3, rats were fed normal diet mixed with EN139505 at 40 ppm (n = 8) or normal diet alone (control, n =10). EN139503 did not affect either food consumption or weight gain.

### Example 12. Feeding studies in weanling pigs.

DON and selected compounds of the invention were tested for their effect *in vivo* in feeding studies conducted in pigs. The pig is the preferred animal model for *in vivo* studies on gut motility as the gut neuromuscular physiology of pigs and humans is similar.

The following protocol was used to study *in vivo* the effect of a compound ("drug") on feed disappearance (food intake) in weanling pigs. In this protocol, mealtimes were restricted to two 45 minutes per day, i.e., in the morning ("AM") and evening ("PM"), and the first meal on Day 1 was preceded by a treat containing a compound ("drugged" or "medicated" treat) of varying doses. All studies were carried out in accordance with the Animal Care Protocol #19970021 issued by the University of Saskatchewan Committee on Animal Care and Supply.

DON, EN139499, and EN139518 were tested at doses of 0.11 mg/kg of body weight (bw) ("low" dose), 0.17 mg/kg bw ("medium" dose) and 0.34 mg/kg bw ("high" dose) of body weight (provided prior to Day 1 AM feeding only, see below). The compounds were administered in a pre-feeding treat consisting of milk replacer top dressed on starter crumbles. The control group received placebo (milk replacer and starter crumbles) treats throughout the Baseline Period (see below) and in the morning (AM) of Day 1 of the test period.

Three separate groups of 20 (one group for each compound) for a total of 60 crossbred (Camborough 15 x Canabrid; Pig Improvement Canada) castrated males (barrows) weighing between 5 and 7 kg and preferable within ± 0.5 kg, 5 ± 3d post-weaning at approximately 25 days of age were selected for each study. Animals that had obvious health problems (e.g., weak, lame, hernia) were excluded from the study. The pigs were identified by ear notching after birth to indicate litter of origin and pig number in the litter. The pigs were weaned at approximately 20 days of age and moved to a production nursery room where they were housed as groups in pens until selection. The pigs were offered *ad libitum* access to a Phase I-medicated nursery diet and water and were managed as per typical nursery procedures until selection.

The zero-dose placebo (control) and the three doses (low, 0.11 mg/kg bw; medium, 0.17 mg/kg bw; and high, 0.34 mg/kg bw) of each compound tested (e.g., DON in Group 1 and other test compound groups) were randomly allocated to five pigs each (using Day -1 weights). Individual pigs housed in individual pens were considered the experimental unit.

The null hypothesis was that pigs offered a test compound would not perform differently from pigs offered the placebo. If the probability was 5% or less that the observed variation among means could occur by chance, the null hypothesis would be rejected and the alternative hypothesis, i.e., that pigs offered a compound in the Day 1 9:00 AM treat performed differently from pigs offered the placebo was accepted.

### Acclimation Period

At the time of selection, the pigs were weighed and moved to individual pens.

The pigs were allowed to become accustomed to the new room and pens, and the lack of penmates for a period of at least 2 days. During this period, the pigs were provided *ad libitum* access to the non-medicated diet and water.

### Baseline Period

Pre-treatment variations in food disappearance and body weight were monitored over five days (Day -5 to Day -1). During this period, the pigs became accustomed to receive and consume a non-medicated (i.e., no compound present) "treat" approximately 20 minutes before the morning (AM) and evening (PM) meals. The pigs were then offered *ad libitum* access to the non-medicated diet for 45 minutes starting at approximately 09:20 AM and 14:20 PM. Pigs were also provided *ad libitum* access to water. Pigs that did not eat their treat within 5 minutes would not receive their meal until 20 minutes after consuming approximately 75% of the treat. Those animals that did not eat their treat did not receive any feed for that feeding time (AM or PM).

### Test Period

A 2-day Test Period followed the Baseline Period (above). For "Day 1 AM", pigs were fed their treat containing the assigned compound (drug) dose mixed in an equivalent amount of feed followed by *ad libitum* access to their meal as described for the Baseline Period (45 minutes only). For "Day 1 PM", pigs were not fed a treat prior to their feed. Likewise, on Day 2 (AM and PM) pigs were not provided a treat prior to being fed. Care was taken to ensure that more than adequate food remained in the feeders at all times during the 45 minute feeding period. Pigs were also provided free access to water. At the end of the each animal's 45 minute feeding period, any feed remaining in the feeder was cleaned up to ensure that each animal had the same feeding time length.

A blood sample was collected from each animal via jugular vena puncture at the end of Day 1 PM 45 minute mealtime. Approximately 5ml of sample were collected in 7 ml red top tubes. These tubes were labelled with abbreviated experiment number, animal tag number, and date.

Within three hours of collection, blood samples were separated by centrifuging for 20 minutes at 3500 x g. The plasma was decanted or aspirated and transferred into a labelled, plastic snap cap tube for later analysis.

Animals were weighed on the morning of Day 3 prior to being removed from the room and returned to the herd.

### Measurements and Observations

Pigs were individually weighed at the time of selection, and daily during the Baseline and Test periods (above) prior to being offered their scheduled AM treat (calculation of dosing for test periods was based on same day weights), and also on Day 3 of the test period before removal from the study. Feed added before and weighed back at the end of each 45 minute mealtime was weighed and recorded. Any spillage was noted and accounted for if substantial.

Individual animal weights were used to calculate daily weight gains for each period and overall (body weight gain per pen divided by the number of days in the specified period).
The amount of feed added and weighed back was used to calculate feed disappearance for each feeding period (AM vs. PM), each day and overall (feed disappearance divided by the number of live pig days in the specified period).

Observations during the first 4 days of Baseline and on Test Day 2 included general statements on animals eating behavior (immediate following presentation or delayed, continuous or intermittent, ending prior to 45 minutes, or food taken while animal still trying to eat, apparent interest in treat and food and mood (playful, tired, anxious)).

Observations on the last day of Baseline and during the Day 1 of the Test Period included when animal started eating treat, when they finished, if they ate treat continuously, if and when emesis (vomiting) occurred, and mood of animal after treat (playful, sleepy, sick, anxious). During the 45 minute feeding period, start and stop times of feed intake were noted, as well as emesis, rooting, mood of animal between eating/drinking (playing, sleeping, sick), an animal's interest in food and treat, and any unusual behavior.

During the study, the pigs in each pen were observed at least two times daily and their health status assessed. Any pig appearing ill were documented and observed more carefully thereafter. Any adverse compound-related effect, other than compound-induced decrease in feed intake, was also documented.

### Diet Formulation, Requirements, Mixing and Sampling

A commercial, non-medicated nursery diet (Ultrawean 21®, Federated CO-OP Limited) was used as the Acclimation, Baseline and Test periods diet. The diet was formulated to provide at least (1.35 % Tlys, 3600 kcal DE) and to exceed the NRC 1998 daily nutrient requirements of 5-12 kg pig. The diet was offered in crumbled form. Feed did not contain detectable levels of any trichothecene as this may confound the study results. Mixtures and calculations used in this study are given below.

### Test Period Day 1 Compound (Drug) Treat Calculations

| Drug Dose Group | Ratio | | Treat Dose Treat Dose |
|---|---|---|---|
| | Drug | Milk Replacer | |
| 0.0 (placebo) | 0 mg | 0.50mg | 0.50mg/kg bw |
| 0.11mg/kg bw | 0.11mg | 0.39mg | 0.50mg/kg bw |
| 0.17mg/kg bw | 0.17mg | 0.33mg | 0.50mg/kg bw |
| 0.34mg/kg bw | 0.34mg | 0.36mg | 0.70mg/kg bw |

### Stock Preparation:

1. Determine the total pig weight per group for Day 1 (5 pigs) = W
2. Multiply this value W x Drug = Total required Drug for given group
3. Multiply this value W x Milk Replacer = Total required milk replacer for given group
4. Thoroughly mix these amounts together in a dose labelled container.

### Determination of each animal's treat:

Multiply each animals weight (in kg) x Treat Dose and weigh out amount into each animal's labelled drug container. Add an equal amount of feed and shake to mix.

### Results

Following the above protocol, the food consumption (normalized) by pigs receiving DON, EN139499, or EN139518 at low, medium, and high concentrations was determined and plotted as bar graphs as shown in Figures 19, 20, and 21, respectively. The data show that all three compounds, provided to each test group in a treat prior to the Day 1 AM meal decreased food consumption by the pigs in the AM meal following the AM treat at one or more of the three doses tested. Furthermore, the effect of each compound on food consumption wore off by the next meal, i.e., the Day 1 PM and following meal times. In fact, some pigs that received medium or high doses of a compound on Day 1 AM appear to have even eaten more in the Day 1 PM meal after the effects of the compound wore off (see, e.g., Day 1-PM for EN139518 treated pigs in Figure 21).

In this study, some emesis occurred in pigs receiving DON at the medium or high doses. However, no emesis occurred in pigs that received EN139499 or EN139518 in the food treat.

The results of this study indicate that compounds of the invention may be used to effectively and temporarily decrease food intake in a desirable, highly controllable manner, e.g., when administered prior to a particular meal, without potential physiological complications that may arise in an individual due to longer term deprivation of nutrient intake.

Furthermore, the *in vivo* data clearly show that the property of decreasing food intake is clearly a separate activity, dissociable from any "toxic" side effect, such as emesis (vomiting), which has historically been viewed as the cause for decreased food intake in animals that have ingested food contaminated with naturally occurring trichothecene compounds such as DON. Particularly interesting in this respect is the data from pigs receiving compounds EN139499 or EN139518, as neither of these compounds of the invention possesses an 8-keto group, which has traditionally been considered critical to the toxic ability of naturally occurring trichothecenes to induce emesis and inhibit protein synthesis.

## Claims

1. A pharmaceutical composition comprising a gut motility regulating compound of formula I: 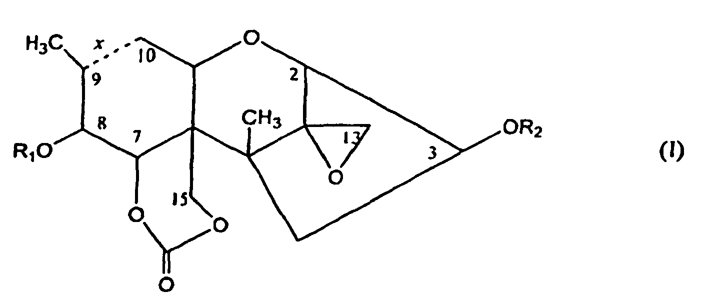 wherein, x is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
a C₁ to C₆ alkyl;
a C₂ to C₆ alkenyl;
a C₂ to C₆ alkynyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; or
a 5- or 6-membered heteroaromatic ring substituted with a substituent selected from the group consisting of halogen, alkyl, alkoxy, thiomethyl, sulfinylmethyl, sulfonylmethyl, and combinations thereof.

2. The pharmaceutical composition comprising a compound according to Claim 1, wherein said compound is selected from the group consisting of 3α-acetoxy-12,13-epoxy-8α-hydroxytrichothec-9-en-7α, 15-carbonate (designated EN 139499); 3α,8α-diacetoxy-12,13-epoxytrichothec-9-en-7α, 15-carbonate (designated EN139500); and 3α,8α-diacetoxy-12,13-epoxy-9α-methyltrichothecan-7α,15-carbonate (designated EN 139507).

3. A pharmaceutical composition comprising a gut motility regulating compound of formula II: 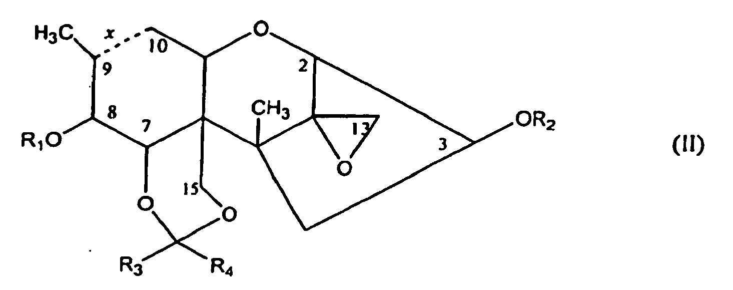 wherein x is a single or double bond between carbon atom-9 and carbon atom- 10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
a C₁ to C₆ alkyl;
a C₂ to C₆ alkenyl;
a C₂ to C₆ alkynyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; or
a 5- or 6-membered heteroaromatic ring substituted with a substituent selected from the group consisting of halogen, alkyl, alkoxy, thiomethyl, sulfinylmethyl, sulfonylmethyl, and combinations thereof;
R₃ and R₄ are, independently:
a hydrogen;
a C₁ to C₆ alkyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.

4. The pharmaceutical composition comprising a compound according to Claim 3, wherein said compound is selected from the group consisting of 3α-acetoxy-7α, 15-benzylidene-12,13-epoxy-9α-methyltrichothecan-8α-ol (designated EN 139501) and 7α, 15-benzylidene-3α,8α-diacetoxy-12,13-epoxy-9α-methyltrichothecane (designated EN 139505).

5. A pharmaceutical composition comprising a gut motility regulating compound of formula III: 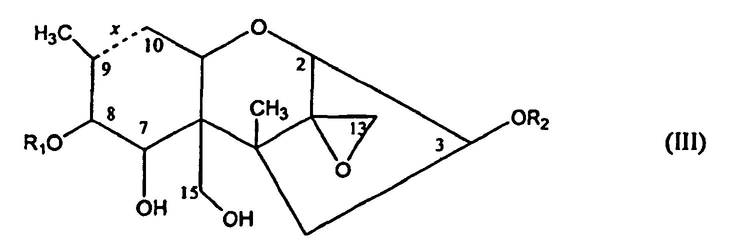 wherein x is a single or double bond between carbon atom-9 and carbon atom-10;
R₁ and R₂ are, independently:
a hydrogen atom;
a C₁ to C₆ alkyl;
a C₁ to C₆ arylalkyl; or
an acyl group C(O)R₅, wherein R₅ is selected from the group consisting of:
a C₁ to C₆ alkyl;
a C₂ to C₆ alkenyl;
a C₂ to C₆ alkynyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring;
a 5- or 6-membered heteroaromatic ring substituted with a substituent selected from the group consisting of halogen, alkyl, alkoxy, thiomethyl, sulfinylmethyl, sulfonylmethyl, and combinations thereof.

6. The pharmaceutical composition comprising a compound according to Claim 5, wherein said compound is selected from the group consisting of 3α-acetoxy-12,13-epoxy-9α-methyltrichothecan-7α,8α,15-triol (designated EN 139503), 3α,8α-diacetoxy-12,13-epoxy-9α-methyltrichothecan-7a,15-diol (designated EN 139506), and 12,13-epoxytrichothec-9-ene-3α,7α,8α, 15-tetraol (designated EN 139518).

7. A pharmaceutical composition comprising a gut motility regulating compound of formula IV: 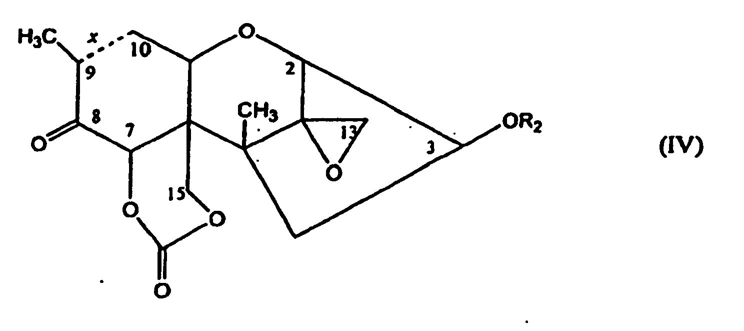 wherein *x* is a single or double bond between carbon atom-9 and carbon atom-10;
R₂ is:
a hydrogen atom;
a C₁ to C₆ alkyl;
a C₁ to C₆ arylalkyl; or
an acyl group C(O)R₅, wherein R₅ is selected from the group consisting of:
a C1 to C6 alkyl;
a C2 to C6 alkenyl;
a C2 to C6 alkynyl;
a phenyl group;
a phenyl group substituted with a substituent selected from the group consisting of halogen, alkyl, thiomethyl, sulfinylmethyl, sulfonylmethyl, and combinations thereof;
a 5- or 6-membered heteroaromatic ring; and
a 5- or 6-membered heteroaromatic ring substituted with a substituent selected from the group consisting of halogen, alkyl, alkoxy, thiomethyl, sulfinylmethyl, sulfonylmethyl, and combinations thereof;
with the proviso that when x is a double bond, then R₂ is not hydrogen or C(O)R5 wherein R₅ is methyl.

8. The pharmaceutical composition comprising a compound according to Claim 7, wherein said compound is selected from the group consisting of 3α-acetoxy-12, 13-epoxy-9α-methyltrichothecan-8-one-7α,15-carbonate (designated EN139511) and 3α-benzoyloxy-12,13-epoxytrichothec-9-en-8-on-7α,15 carbonate (designated EN139517).

9. A pharmaceutical composition comprising a gut motility-regulating compound of formula V: 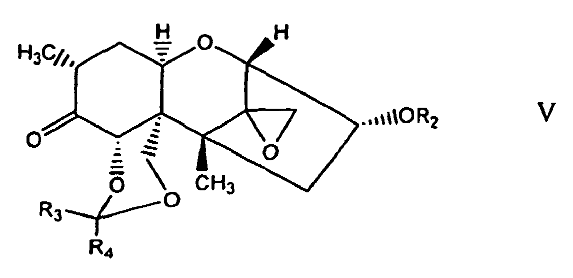 wherein R₂ is:
a hydrogen atom;
a C₁ to C₆ alkyl;
a C₁ to C₆ arylalkyl; or
an acyl group C(O)R₅, wherein R₅ is selected from the group consisting of:
a C₁ to C₆ alkyl;
a C₂ to C₆ alkenyl;
a C₂ to C₆ alkynyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; and
a 5- or 6-membered heteroaromatic ring substituted with a substituent selected from the group consisting of halogen, alkyl, alkoxy, thiomethyl, sulfinylmethyl, sulfonylmethyl, and combinations thereof; and
R₃ and R₄ are, independently:
a hydrogen;
a C₁to C₆ alkyl;
a phenyl group;
a phenyl group substituted with a substituent selected from the group consisting of
halogen, alkyl, alkoxy, thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.

10. The pharmaceutical composition comprising a compound according to Claim 9, wherein said compound is selected from the group consisting of 3-α-acetoxy-7α, I 5-benzylidene-12,13-epoxytrichopthecan-8-one (designated EN 139519) and 7α, 15-benzylidene-12,13-epoxy-3β-hydroxytrichothecan-8-one (designated EN139520).

11. A pharmaceutical composition comprising a gut motility-regulating compound of formula VI: 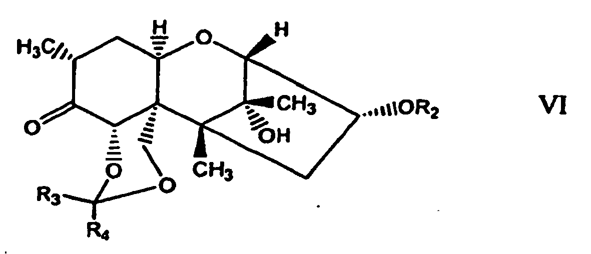 wherein R₂ is:
a hydrogen atom;
a C₁ to C₆ alkyl;
a C₁ to C₆ arylalkyl; or
an acyl group C(O)R₅, wherein R₅ is selected from the group consisting of:
a C₁ to C₆ alkyl;
a C₂ to C₆ alkenyl;
a C₂ to C₆ alkynyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; and
a 5- or 6-membered heteroaromatic ring substituted with a substituent selected from the group consisting of halogen, alkyl, alkoxy, thiomethyl, sulfinylmethyl, sulfonylmethyl, and combinations thereof; and
R₃ and R₄ are, independently:
a hydrogen;
a C₁ to C₆ alkyl;
a phenyl group;
a phenyl group substituted with a substituent selected from the group consisting of halogen, alkyl, alkoxy, thiomethyl, sulfinylmethyl, sulfonylmethyl, and combinations thereof;
a 5- or 6-membered heteroaromatic ring; or
R₃ and R₄, together with the acetal carbon atom, form a carbocyclic ring having 5, 6, or 7 carbon atoms.

12. The pharmaceutical composition comprising a compound according to Claim 11, wherein said compound is 7α,15-benzylidene-9α,12β-dimethyl-3α,12α-dihydroxytrichothecan-8-one (designated EN139522).

13. A pharmaceutical composition comprising 9α,12β-dimethyltrichothecan-3α,7α,8α,12α,15-pentaol (designated as EN 139504).

14. A pharmaceutical composition comprising 7α,15-benzylidene-3α,8α-diacetoxy-9α,12β-dimethyltrichothecan-12α-ol (designated EN139508).

15. A pharmaceutical composition for enhancing or intensifying the fasting pattern of gut motility comprising 7α,15-benzylidene-9α,12β-methyltrichothecan-3α,8α,12α-triol (designated EN139502).

16. A pharmaceutical composition according to any one of Claims 1 to 15, further comprising a pharmaceutically acceptable carrier.

17. A compound of formula (I) as defined in claim 1 or 2, formula (II) as defined in claim 3 or 4, formula (III) as defined in claim 5 or 6, formula (IV) as defined in claim 7 or 8, formula (V) as defined in claim 9 or 10, formula (VI) as defined in claim 11 or 12 or a compound as defined in claim 13, 14 or 15, with the provisos that a compound of formula (III) as defined in claim 5 is not the compound wherein simultaneously x is a double bond, R₁ is hydrogen and R₂ is C(O)R₅ wherein R₅ is methyl or the compound wherein simultaneously x is a double bond, R₁ is hydrogen and R₂ is hydrogen, and that a compound as defined in claim 6 is not 12, 13-epoxytrichothec-9-ene-3α, 7α, 8a, 15-tetra ol (designated EN 139518).

18. A compound of formula (I) as defined in claim 1 or 2, formula (II) as defined in claim 3 or 4, formula (III) as defined in claim 5 or 6, formula (IV) as defined in claim 7 or 8, formula (V) as defined in claim 9 or 10, formula (VI) as defined in claim 11 or 12 or a compound as defined in claim 13 or 14 for use as a medicament.

19. A compound as claimed in claim 18 for use as a medicament for reducing food intake in an individual.

20. A compound as claimed in claim 18 for use as a medicament for treating obesity.

21. A compound as claimed in claim 18 for use as a medicament for inducing fed pattern of gut motility in an individual.

22. Use of a compound as claimed in claim 18 or a composition as claimed in any one of claims 1 to 14 for the manufacture of a medicament for reducing food intake in an individual.

23. Use of a compound as claimed in claim 18 or a composition as claimed in any one of claims 1 to 14 for the manufacture of a medicament for treating obesity.

24. Use of a compound as claimed in claim 18 or a composition as claimed in any one of claims 1 to 14 for the manufacture of a medicament for inducing fed pattern of gut motility in an individual.

25. The use according to any one of claims 22 to 24, wherein said medicament is for oral administration.

26. A compound as defined in claim 15 for use as a medicament.

27. A compound as claimed in claim 26 for use as a medicament for increasing food intake in an individual.

28. A compound as claimed in claim 26 for use as a medicament for treating malnutrition.

29. A compound as claimed in claim 26 for use as a medicament treating anorexia.

30. Use of a compound as claimed in claim 26 or a composition as claimed in claim 15 for the manufacture of a medicament for increasing food intake in an individual.

31. Use of a compound as claimed in claim 26 or a composition as claimed in claim 15 for the manufacture of a medicament for treating malnutrition.

32. Use of a compound as claimed in claim 26 or a composition as claimed in claim 15 for the manufacture of a medicament for treating anorexia.

33. The use according to any one of claims 30 to 32, wherein said medicament is for oral administration.

## Patentansprüche

1. Eine pharmazeutische Zusammensetzung, beinhaltend eine die Darmtätigkeit regulierende Verbindung der Formel I: 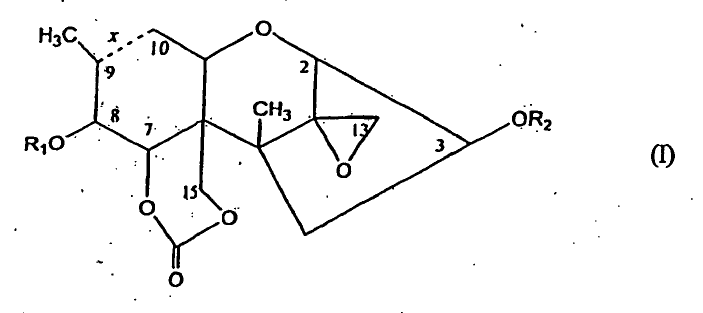 worin x eine Einfach- oder eine Doppelbindung zwischen Kohlenstoffatom 9 und Kohlenstoffatom 10 ist;
R₁ und R₂ unabhängig voneinander
ein Wasserstoffatom;
ein C₁- bis C₆-Alkyl;
ein C₁- bis C₆-Arylalkyl; oder
eine Acylgruppe C(O)R₅ sind, wobei R₅ ausgewählt ist aus der Gruppe bestehend aus:
einem C₁- bis C₆-Alkyl;
einem C₂- bis C₆-Alkenyl;
einem C₂- bis C₆-Alkynyl;
einer Phenylgruppe;
einer Phenylgruppe, die mit einem Substituenten substituiert ist, der ausgewählt ist aus der Gruppe bestehend aus Halogen, Alkyl, Alkoxy, Thiomethyl, Sulfinymethyl, Sulfonylmethyl und deren Kombinationen;
einem 5- oder 6-gliedrigen heteroaromatischen Ring; oder
einem 5- oder 6-gliedrigen heteroaromatischen Ring substituiert mit einem Substituenten, der ausgewählt ist aus der Gruppe bestehend aus Halogen, Alkyl, Alkoxy, Thiomethyl, Sulfinylmethyl, Sulfonylmethyl und deren Kombinationen.

2. Die pharmazeutische Zusammensetzung, beinhaltend eine Verbindung nach Anspruch 1, wobei die Verbindung ausgewählt ist aus der Gruppe bestehend aus 3α-Acetoxy-12,13-epoxy-8α-hydroxytrichotheo-9-en-7α, 15-carbonat (bezeichnet als EN139499); 3α,8α-Diacetoxy-12,13-epoxytrichothec-9-en-7α,15-carbonat (bezeichnet als EN139500); und 3α,8α-Diacetoxy 12,13-epoxy-9α-methyltrichothecan-7α,15-carbonat (bezeichnet als EN139507).

3. Eine pharmazeutische Zusammensetzung, beinhaltend eine die Darmtätigkeit regulierende Verbindung der Formel II: 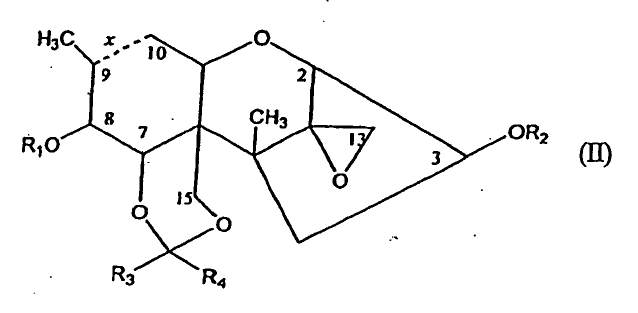 wobei x eine Einfach- oder Doppelbindung zwischen dem Kohlenstoffatom 9 und dem Kohlenstoffatom 10 ist;
R₁ und R₂ unabhängig voneinander:
ein Wasserstoffatom;
ein C₁- bis C₆-Alkyl;
ein C₁- bis C₆-Arylalkyl; oder
eine Acylgruppe C(O)R₅ ist, wobei R₅ ausgewählt ist aus der Gruppe bestehend aus:
einem C₁ - bis C₆-Alkyl;
einem C₂- bis C₆-Alkenyl;
einem C₂- bis C₆-Alkynyl;
einer Phenylgruppe;
einer Phenylgruppe, die mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen;
einem 5- oder 6-heteroaromatischen Ring; oder
einem 5- oder 6-gliedrigen heteroaromatischen Ring substituiert mit einem Substituenten, der ausgewählt ist aus der Gruppe bestehend aus Halogen, Alkyl, Alkoxy, Thiomethyl, Sulfinylmethyl, Sulfonylmethyl und deren Kombinationen;
R₃ und R₄ unabhängig voneinander:
ein Wasserstoffatom;
ein C₁- bis C₆-Alkyl;
eine Phenylgruppe;
eine Phenylgruppe sind, substituiert mit einem Substituenten, der ausgewählt ist aus der Gruppe bestehend aus Halogen, Alkyl, Alkoxy, Thiomethyl, Sulfinylmethyl, Sulfonylmethyl und deren Kombinationen;
ein 5- oder 6-gliedriger heteroaromatischer Ring, sind oder
R₃ und R₄ zusammen mit dem Acetalkohlenstoffatom einen 5, 6 oder 7 Kohlenstoffatome aufweisenden carbozyklischen Ring bilden.

4. Die pharmazeutische Zusammensetzung, beinhaltend eine Verbindung nach Anspruch 3, wobei die Verbindung ausgewählt ist aus der Gruppe bestehend aus 3α-Acetoxy-7α,15-benzyliden-12,13-epoxy-9α-methyltrichothecan-8α-ol (bezeichnet als EN139501) und 7α,15-Benzyliden-3α,8α-diacetoxy-12,13-epoxy-9α-methyltrichothecan (bezeichnet als EN139505).

5. Eine pharmazeutische Zusammensetzung, beinhaltend eine die Darmtätigkeit regulierende Verbindung der Formel III: 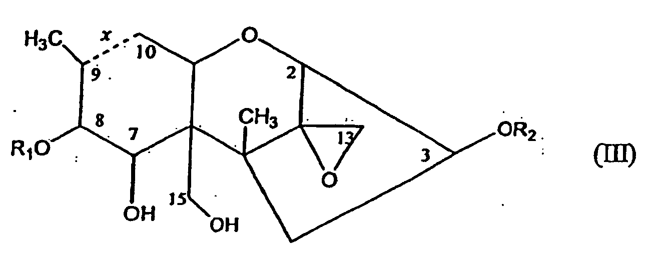 wobei x eine Einfach- oder eine Doppelbindung zwischen Kohlenstoffatom 9 und Kohlenstoffatom 10 ist;
R₁ und R₂ unabhängig voneinander
ein Wasserstoffatom;
ein C₁- bis C₆-Alkyl;
ein C₁- bis C₆-Arylalkyl; oder
eine Acylgruppe C(O)R₅ sind, wobei R₅ ausgewählt ist aus der Gruppe bestehend aus:
einem C₁- bis C₆-Alkyl;
einem C₂- bis C₆-Alkenyl;
einem C₂- bis C₆-Alkynyl;
einer Phenylgruppe;
einer Phenylgruppe, die mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen;
einem 5- oder 6-gliedrigen heteroaromatischen Ring;
einem 5- oder 6-gliedrigen heteroaromatischen Ring substituiert mit einem Substituenten, der ausgewählt ist aus der Gruppe bestehend aus Halogen, Alkyl, Alkoxy, Thiomethyl, Sulfinylmethyl, Sulfonylmethyl und deren Kombinationen.

6. Die pharmazeutische Zusammensetzung, beinhaltend eine Verbindung nach Anspruch 5, wobei die Verbindung ausgewählt ist aus der Gruppe bestehend aus 3α-Acetoxy-12,13-epoxy-9α-methyltrichothecan-7α,8α,15-triol (bezeichnet als EN139503), 3α,8α-Diacetoxy-12,13-epoxy-9α-methyltrichothecan-7α,15-diol (bezeichnet als EN139506) und 12,13-Epoxytrichothec-9-en-3α,7α, 8α, 15-tetraol (bezeichnet als EN139518).

7. Eine pharmazeutische Zusammensetzung, beinhaltend eine die Darmtätigkeit regulierende Verbindung der Formel IV: 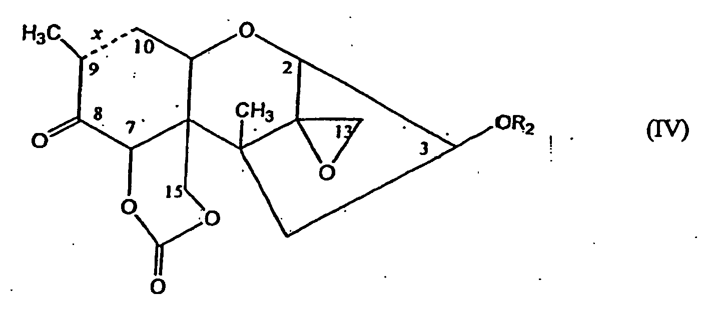 wobei x eine Einfach- oder eine Doppelbindung zwischen Kohlenstoffatom 9 und Kohlenstoffatom10 ist;
R₂
ein Wasserstoffatom;
ein C₁- bis C₆-Alkyl;
ein C₁- bis C₆-Arylalkyl; oder
eine Acylgruppe C(O)R₅ ist, wobei R₅ ausgewählt ist aus der Gruppe
bestehend aus:
einem C₁- bis C₆-Alkyl;
einem C₂- bis C₆-Alkenyl;
einem C₂- bis C₆-Alkynyl;
einer Phenylgruppe;
einer Phenylgruppe, die mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen;
einem 5- oder 6-gliedrigen heteroaromatischen Ring; und
einem 5- oder 6-gliedrigen heteroaromatischen Ring, der mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen;
mit der Vorgabe, dass wenn x eine Doppelbindung ist, R₂ dann kein Wasserstoff oder C(O)R₅ ist, wobei R₅ Methyl ist.

8. Die pharmazeutische Zusammensetzung, beinhaltend eine Verbindung nach Anspruch 7, wobei die Verbindung ausgewählt ist aus der Gruppe bestehend aus 3α-Acetoxy-12,13-epoxy-9α-methyltrichothecan-8-on-7α,15-carbonat (bezeichnet als EN139511) und 3α-Benzoyloxy-12,13-epoxykrichothec-9-en-8-on-7α,15-carbonat (bezeichnet als EN139514).

9. Eine pharmazeutische Zusammensetzung, beinhaltend eine die Darmtätigkeit regulierende Verbindung der Formel V: 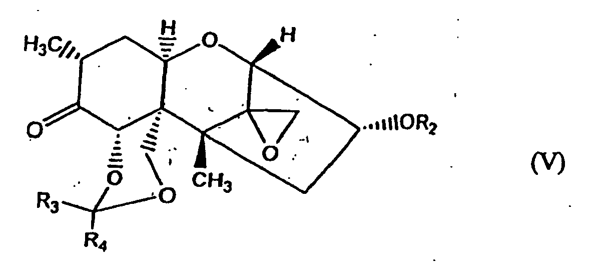 wobei R₂
ein Wasserstoffatorn;
ein C₁- bis C₆-Alkyl;
ein C₁- bis C₆-Arylalkyl; oder
eine Acylgruppe C(O)R₅ sind, wobei R₅ ausgewählt ist aus der Gruppe bestehend aus:
einem C₁- bis C₆-Alkyl;
einem C₂- bis C₆-Alkenyl;
einem C₂- bis C₆-Alkynyl;
einer Phenylgruppe;
einer Phenylgruppe, die mit einem Substituenten substituiert ist, der ausgewählt ist aus der Gruppe bestehend aus Halogen, Alkyl, Alkoxy, Thiomethyl, Sulfinylinethyl, Sulfonylmethyl und deren Kombinationen;
einem 5- oder 6-gliedrigen heteroaromatischen Ring; und
einem 5- oder 6-gliedrigen heteroaromatischen Ring, der mit einem Substituenten substituiert ist, der ausgewählt ist aus der Gruppe bestehend
aus Halogen, Alkyl, Alkoxy, Thiomethyl, Sulfinylmethyl, Sulfonylmethyl und deren Kombinationen; und
R₃ und R₄ unabhängig voneinander
ein Wasserstoff;
ein C₁- bis C₆-Alkyl;
eine Phenylgruppe;
eine Phenylgruppe sind, die mit einem Substituenten substituiert ist, ausgewählt aus der Gruppe bestehend aus Halogen, Alkyl, Alkoxy, Thiomethyl, Sulfinylmethyl, Sulfonylmethyl und deren Kombinationen;
ein 5- oder 6-gliedrigen heteroaromatischen Ring; oder
R₃ und R₄ bilden einen carbozyklischen Ring zusammen mit einem Acetalkohlenstoffatom, der 5, 6 oder 7 Kohlenstoffatome hat.

10. Die pharmazeutische Zusammensetzung, beinhaltend eine Verbindung nach Anspruch 9, wobei die Verbindung ausgewählt ist aus der Gruppe bestehend aus 3-α-Acetoxy-7α,15-benzyliden-12,13-epoxytrichothecan-8-on (bezeichnet als EN139519) und 7α,15-Benzyliden-12,13-epoxy-3β-hydroxytrichothecan-8-on (bezeichnet als EN139520).

11. Eine pharmazeutische Zusammensetzung, beinhaltend eine die Darmtätigkeit regulierende Verbindung der Formel VI: 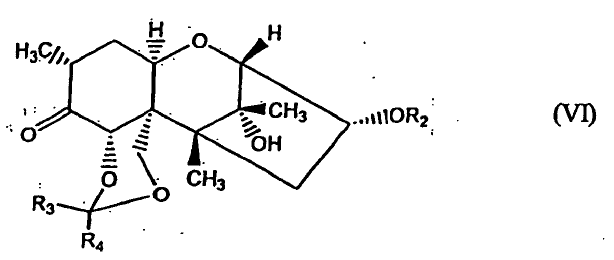 wobei R₂:
ein Wasserstoffatom;
ein C₁bis C₆-Alkyl;
ein C₁ bis C₆ Arylalkyl; oder
eine Acylgruppe C(O)R₅ ist, wobei R₅ ausgewählt ist aus der Gruppe bestehend aus:
einem C₁- bis C₂-Alkyl;
einem C₂- zu C₆-Alkenyl;
einem C₂- zu C₆-Alkynyl;
einer Phenylgruppe;
einer Phenylgruppe, die mit einem Substituenten substituiert ist, ausgewählt aus der Gruppe bestehend aus Halogen, Alkyl, Alkoxy, Thiomethyl, Sulfinylmethyl, Sulfonylmethyl sowie deren Kombinationen;
einem 5- oder 6-gliedrigen heteroaromatischen Ring, und
einem 5- oder 6-heteroaromatischen Ring, der mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen; und
R₃ und R₄ unabhängig voneinander:
ein Wasserstoff;
ein C₁- bis C₆-Alkyl;
eine Phenylgruppe;
eine Phenylgruppe, die mit einem Substituenten substituiert ist, der ausgewählt ist aus der Gruppe bestehend aus Halogen, Alkyl, Alkoxy, Thiomethyl, Sulfinylmethyl, Sulfonylmethyl und deren Kombinationen;
ein 5- oder 6-gliedriger heteroaromatischer Ring sind; oder
R₃ und R₄ zusammen mit dem Acetalkohlenstoffatom einen carbocyclischen Ring bilden, der 5, 6 oder 7 Kohlenstoffatome hat.

12. Die pharmazeutische Zusammensetzung, beinhaltend eine Verbindung nach Anspruch 11, wobei die Verbindung 7α,15-Benzyliden-9α,12β-dimethyl-3α,12α-dihydroxytrichothecan-8-on (bezeichnet als EN139522) ist.

13. Eine pharmazeutische Zusammensetzung, beinhaltend 9α,12β-Dimethyltrichothecan-3α,7α,8α,12α,15-pentaol (bezeichnet als EN139504).

14. Eine pharmazeutische Zusammensetzung, beinhaltend 7α,15-Benzyliden-3α,8α-diacetoxy-9α,12β-dimethyltrichothecan-12α-ol (bezeichnet als EN139508).

15. Eine pharmazeutische Zusammensetzung, zum Verbessern oder zum Verstärken des Fastenmusters im Hinblick auf die Darmtätigkeit, beinhaltend 7α,15-Benzyliden-9α,12β-methyltrichothecan-3α,8α,12α-triol (bezeichnet als EN139502).

16. Eine pharmazeutische Zusammensetzung nach einem der Ansprüche 1 bis 15, weiterhin beinhaltend einen pharmazeutisch akzeptablen Träger.

17. Eine Verbindung der Formel (I), wie in Anspruch 1 oder 2 definiert, der Formel (II) wie in Anspruch 3 oder 4 definiert, der Formel (III) wie in Anspruch 5 oder 6 definiert, der Formel (IV) wie in Anspruch 7 oder 8 definiert, der Formel (V) wie in Anspruch 9 oder 10 definiert, der Formel (VI) wie in Anspruch 11 oder 12 definiert oder eine Verbindung wie in Anspruch 13, 14 oder 15 definiert, mit den Vorgaben, dass eine Verbindung der Formel (III), wie in Anspruch 5 definiert, nicht die Verbindung ist, in der x gleichzeitig eine Doppelbindung ist, R₁ Wasserstoff und R₂ C(O)R₅ ist, wobei R₅ Methyl ist oder die Verbindung, in der x gleichzeitig eine Doppelbindung ist, R₁ Wasserstoff und R₂ Wasserstoff ist und dass eine Verbindung, wie in Anspruch 6 definiert, nicht 12,13-Epoxytrichothec-9-en-3α, 7α,8α,15-tetra ol (bezeichnet als EN I39518) ist.

18. Eine Verbindung der Formel (I), wie in Anspruch 1 oder 2 definiert, der Formel (II), wie in Anspruch 3 oder 4 definiert, der Formel (III) wie in Anspruch 5 oder 6 definiert, der Formel (IV) wie in Anspruch 7 oder 8 definiert, der Formel (V) wie in Anspruch 9 oder 10, definiert, der Formel (IV) wie in Anspruch 11 oder 12 definiert oder eine Verbindung wie in Anspruch 13 oder 14 definiert zur Verwendung als ein Medikament.

19. Eine Verbindung nach Anspruch 18 zur Verwendung als ein Medikament zur Reduzierung der Nahrungsaufnahme bei einer Person.

20. Eine Verbindung nach Anspruch 18 zur Anwendung als ein Medikament zur Behandlung der Fettleibigkeit.

21. Eine Verbindung nach Anspruch 18 zur Anwendung als ein Medikament zum Induzieren eines Fütterungsmusters der Darmtätigkeit bei einer Person.

22. Verwendung einer Verbindung nach Anspruch 18 oder einer Zusammensetzung nach einem der Ansprüche 1 bis 14 zur Herstellung eines Medikaments zur Reduzierung der Nahrungsaufnahme bei einer Person.

23. Verwendung einer Verbindung nach Anspruch 18 oder einer Zusammensetzung nach einem der Ansprüche 1 bis 14 zur Herstellung eines Medikaments zur Behandlung von Fettleibigkeit.

24. Verwendung einer Verbindung nach Anspruch 18 oder einer Zusammensetzung nach einem der Ansprüche 1 bis 14 zur Herstellung eines Medikaments zum Induzieren eines Fütterungsmusters der Darmtätigkeit bei einer Person.

25. Die Verwendung nach einem der Ansprüche 22 bis 24, wobei das Medikament zur oralen Verabreichung dient.

26. Eine Verbindung nach Anspruch 15 zur Verwendung als ein Medikament.

27. Eine Verbindung nach Anspruch 26 zur Verwendung als ein Medikament zur Erhöhung der Nahrungsaufnahme bei einer Person.

28. Eine Verbindung nach Anspruch 26 zur Verwendung als ein Medikament zur Behandlung von Mangelemährung.

29. Eine Verbindung nach Anspruch 26 zur Verwendung als ein Medikament zur Behandlung von Anorexie.

30. Verwendung einer Verbindung nach Anspruch 26 oder einer Zusammensetzung nach Anspruch 15 zur Herstellung eines Medikaments zur Erhöhung der Nahrungsaufnahme bei einer Person.

31. Verwendung einer Verbindung nach Anspruch 26 oder einer Zusammensetzung nach Anspruch 15 zur Herstellung eines Medikaments zur Behandlung von Mangelernährung.

32. Verwendung einer Verbindung nach Anspruch 26 oder einer Zusammensetzung nach Anspruch 15 zur Herstellung eines Medikaments zur Behandlung von Anorexie.

33. Die Verwendung nach einem der Ansprüche 30 bis 32, wobei das Medikament zur oralen Verabreichung dient.

## Revendications

1. Composition pharmaceutique comprenant un composé qui régule le transit intestinal de formule (I) : 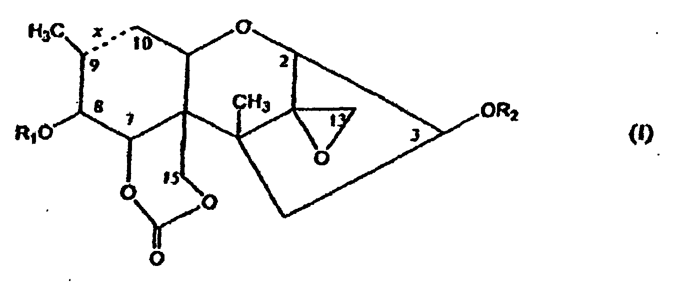 dans laquelle :
x est une liaison simple ou double située entre l'atome de carbone en 9 et l'atome de carbone en 10 ;
R₁ et R₂ sont indépendamment :
un atome d'hydrogène ;
un groupe alkyle en C₁₋₆;
un groupe arylalkyle en C₁₋₆ ; ou
un groupe acyle C(O)R₅, dans lequel R₅ est choisi dans le groupe consistant en :
un groupe alkyle en C₁₋₆;
un groupe alcényle en C₂₋₆ ;
un groupe alcynyle en C₂₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons.

2. Composition pharmaceutique comprenant un composé selon la revendication 1, dans laquelle ledit composé est choisi dans le groupe consistant en 3α-acétoxy-12,13-époxy-8α-hydroxy-trichothéc-9-èn-7α, 15-carbonate (désigné EN139499), 3α,8α-diacétoxy-12,13-époxytrichothéc-9-èn-7α,15-carbonate (désigné EN 139500) et 3α,8α-diacétoxy-12,13-époxy-9α-méthyltrichothécan-7α,15-carbonate (désigné EN139507).

3. Composition pharmaceutique comprenant un composé qui régule le transit intestinal de formule (II) : 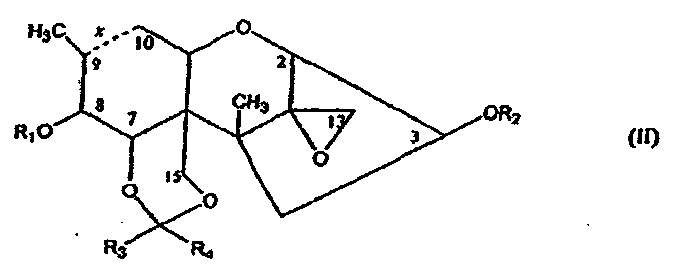 dans laquelle :
x est une liaison simple ou double située entre l'atome de carbone en 9 et l'atome de carbone en 10 ;
R₁ et R₂ sont indépendamment :
un atome d'hydrogène;
un groupe alkyle en C₁₋₆;
un groupe arylalkyle en C₁₋₆; ou
un groupe acyle C(O)R₅, dans lequel R₅ est choisi dans le groupe consistant en :
un groupe alkyle en C₁₋₆;
un groupe alcényle en C₂₋₆;
un groupe alcynyle en C₂₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons ;
R₃ et R₄ sont indépendamment :
un atome d'hydrogène ;
un groupe alkyle en C₁₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou bien
R₃ et R₄ forment ensemble avec l'atome de carbone de l'acétal, un noyau carbocyclique ayant 5, 6 ou 7 atomes de carbone.

4. Composition pharmaceutique comprenant un composé selon la revendication 3, dans laquelle ledit composé est choisi dans le groupe consistant en 3α-acétoxy-7α, 15-benzylidène-12,13-époxy-9α-méthyltrichothécan-8α-ol (désigné EN139501) et 7α, 15-benzylidène-3α,8α-diacétoxy-12,13-époxy-9α-méthyltrichothécane (désigné EN139505).

5. Composition pharmaceutique comprenant un composé qui régule le transit intestinal de formule (III) : 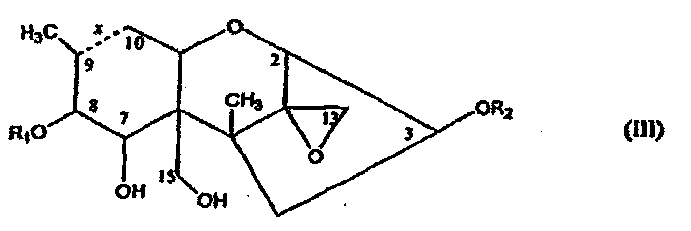 dans laquelle :
x est une liaison simple ou double située entre l'atome de carbone en 9 et l'atome de carbone en 10 ;
R₁ et R₂ sont indépendamment :
un atome d'hydrogène ;
un groupe alkyle en C₁₋₆ ;
un groupe arylalkyle en C₁₋₆ ; ou
un groupe acyle C(O)R₅, dans lequel R₅ est choisi dans le groupe consistant en :
un groupe alkyle en C₁₋₆;
un groupe alcényle en C₂₋₆;
un groupe alcynyle en C₂₋₆ ;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons.

6. Composition pharmaceutique comprenant un composé selon la revendication 5, dans laquelle ledit composé est choisi dans le groupe consistant en 3α-acétoxy-12,13-époxy-9α-méthyl-trichothécan-7α,8α,15-triol (désigné EN139503), 3α,8α-diacétoxy-12,13-époxy-9α-méthyltrichothécan-7α,15-diol (désigné EN139506) et 12,13-époxytrichothéc-9-èn-3α,7α,8α,15-tétraol (désigné EN139518).

7. Composition pharmaceutique comprenant un composé qui régule le transit intestinal de formule (IV) : 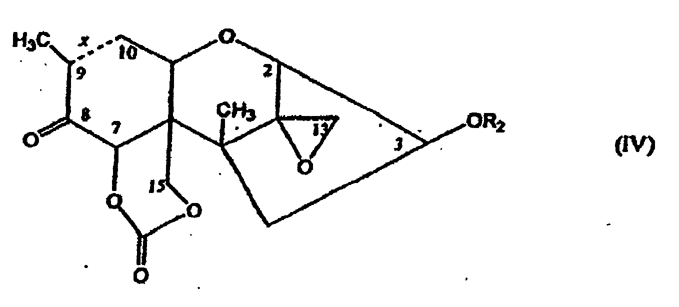 dans laquelle :
x est une liaison simple ou double située entre l'atome de carbone en 9 et l'atome de carbone en 10 ;
R₂ est:
un atome d'hydrogène ;
un groupe alkyle en C₁₋₆;
un groupe arylalkyle en C₁₋₆; ou
un groupe acyle C(O)R₅, dans lequel R₅ est choisi dans le groupe consistant en :
un groupe alkyle en C₁₋₆;
un groupe alcényle en C₂₋₆;
un groupe alcynyle en C₂₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; et
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons ;
à la condition que lorsque x est une double liaison, alors R₂ n'est pas un atome d'hydrogène ou un groupe C(O)R₅ dans lequel R₅ est un groupe méthyle.

8. Composition pharmaceutique comprenant un composé selon la revendication 7, dans laquelle ledit composé est choisi dans le groupe consistant en 3α-acétoxy-12,13-époxy-9α-méthyl-trichothécan-8-one-7α, 15-carbonate (désigné EN139511) et 3α-benzoyloxy-12,13-époxytrichothéc-9-èn-8-on-7α,15-carbonate (désigné EN139514).

9. Composition pharmaceutique comprenant un composé qui régule le transit intestinal de formule (V) : 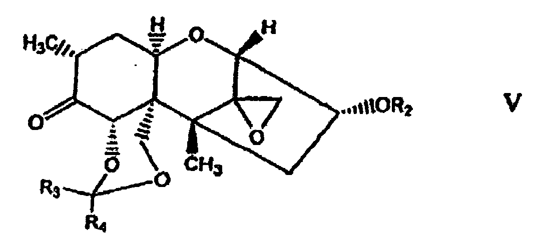 dans laquelle :
R₂ est:
un atome d'hydrogène ;
un groupe alkyle en C₁₋₆;
un groupe arylalkyle en C₁₋₆; ou
un groupe acyle C(O)R₅, dans lequel R₅ est choisi dans le groupe consistant en :
un groupe alkyle en C₁₋₆;
un groupe alcényle en C₂₋₆;
un groupe alcynyle en C₂₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; et
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons ; et
R₃ et R₄ sont indépendamment :
un atome d'hydrogène ;
un groupe alkyle en C₁₋₆ ;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou bien
R₃ et R₄ forment ensemble avec l'atome de carbone de l'acétal, un noyau carbocyclique ayant 5, 6 ou 7 atomes de carbone.

10. Composition pharmaceutique comprenant un composé selon la revendication 9, dans laquelle ledit composé est choisi dans le groupe consistant en 3α-acétoxy-7α,15-benzylidène-12,13-époxy-trichothécan-8-one (désigné EN139519) et 7α,15-benzylidène-12,13-époxy-3β-hydroxytrichothécan-8-one (désigné EN139520).

11. Composition pharmaceutique comprenant un composé qui régule le transit intestinal de formule (VI) : 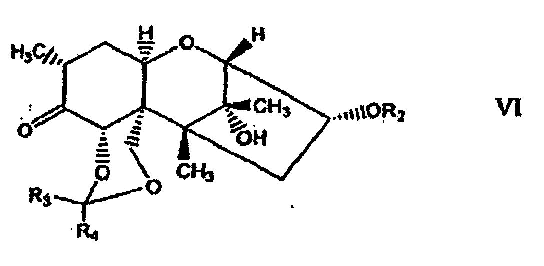 dans laquelle :
R₂ est:
un atome d'hydrogène ;
un groupe alkyle en C₁₋₆ ;
un groupe arylalkyle en C₁₋₆ ; ou
un groupe acyle C(O)R₅, dans lequel R₅ est choisi dans le groupe consistant en :
un groupe alkyle en C₁₋₆;
un groupe alcényle en C₂₋₆;
un groupe alcynyle en C₂₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; et
un cycle hétéroaromatique de 5 ou 6 chaînons substitué par un substituant choisi dans le groupe consistant en atome d'halogène, groupe alkyle, alcoxy, thiométhyle, sulfinylméthyle, sulfonylméthyle et leurs combinaisons ; et
R₃ et R₄ sont indépendamment :
un atome d'hydrogène ;
un groupe alkyle en C₁₋₆;
un groupe phényle ;
un groupe phényle substitué par un substituant choisi dans le groupe consistant en atome d'halogène, groupe alkyle, alcoxy, thiométhyle, sulfinylméthyle, sulfonylméthyle et leurs combinaisons ;
un cycle hétéroaromatique de 5 ou 6 chaînons ; ou
R₃ et R₄ forment ensemble avec l'atome de carbone de l'acétal, un noyau carbocyclique ayant 5, 6 ou 7 atomes de carbone.

12. Composition pharmaceutique comprenant un composé selon la revendication 11, dans laquelle ledit composé est la 7α,15-benzylidène-9α,12β-diméthyl-3α,12α-dihydroxytrichothécan-8-one (désigné EN139522).

13. Composition pharmaceutique comprenant le 9α,12β-diméthyltricho-thécan-3α,7α,8α,12α,15-pentaol (désigné EN139504).

14. Composition pharmaceutique comprenant le 7α,15-benzylidène-3α,8α-diacétoxy-9α,12β-diméthyltrichothécan-12α-ol (désigné EN139508).

15. Composition pharmaceutique pour accroître ou intensifier le régime de jeûne du transit intestinal comprenant le 7α,15-benzylidène-9α,12β-méthyltri-chothécan-3α,8α,12α-triol (désigné EN139502).

16. Composition pharmaceutique selon l'une quelconque des revendications 1 à 15, comprenant de plus un support acceptable du point de vue pharmaceutique.

17. Composé de formule (I) selon la revendication 1 ou 2, de formule (II) selon la revendication 3 ou 4, de formule (III) selon la revendication 5 ou 6, de formule (IV) selon la revendication 7 ou 8, de formule (V) selon la revendication 9 ou 10, de formule (VI) selon la revendication 11 ou 12, ou composé selon les revendications 13, 14 ou 15, à condition qu'un composé de formule (III) selon la revendication 5 ne soit pas le composé dans lequel simultanément x est une double liaison, R₁ est un atome d'hydrogène et R₂ est un groupe C(O)R₅ dans lequel R₅ est un groupe méthyle, ou le composé dans lequel simultanément x est une double liaison, R₁ est un atome d'hydrogène et R₂ est un atome d'hydrogène, et qu'un composé selon la revendication 6 ne soit pas le 12,13-époxytrichothéc-9-èn-3α,7α,8α,15-tétraol (désigné EN139518).

18. Composé de formule (I) selon la revendication 1 ou 2, de formule (II) selon la revendication 3 ou 4, de formule (III) selon la revendication 5 ou 6, de formule (IV) selon la revendication 7 ou 8, de formule (V) selon la revendication 9 ou 10, de formule (VI) selon la revendication 11 ou 12, ou composé selon les revendications 13 ou 14 utile en tant que médicament.

19. Composé selon la revendication 18, utile en tant que médicament destiné à réduire la ration alimentaire chez un individu.

20. Composé selon la revendication 18, utile en tant que médicament destiné à traiter l'obésité.

21. Composé selon la revendication 18, utile en tant que médicament destiné à induire un régime alimenté de transit intestinal chez un individu.

22. Utilisation d'un composé selon la revendication 18 ou d'une composition selon l'une quelconque des revendications 1 à 14, pour la fabrication d'un médicament destiné à réduire la ration alimentaire chez un individu.

23. Utilisation d'un composé selon la revendication 18 ou d'une composition selon l'une quelconque des revendications 1 à 14, pour la fabrication d'un médicament destiné à traiter l'obésité.

24. Utilisation d'un composé selon la revendication 18 ou d'une composition selon l'une quelconque des revendications 1 à 14, pour la fabrication d'un médicament destiné à induire un régime alimenté de transit intestinal chez un individu.

25. Utilisation selon l'une quelconque des revendications 22 à 24, dans laquelle ledit médicament est destiné à l'administration orale.

26. Composé selon la revendication 15, utile en tant que médicament.

27. Composé selon la revendication 26, utile en tant que médicament destiné à augmenter la ration alimentaire chez un individu.

28. Composé selon la revendication 26, utile en tant que médicament destiné à traiter la malnutrition.

29. Composé selon la revendication 26, utile en tant que médicament destiné à traiter l'anorexie.

30. Utilisation d'un composé selon la revendication 26 ou d'une composition selon la revendication 15, pour la fabrication d'un médicament destiné à augmenter la ration alimentaire chez un individu.

31. Utilisation d'un composé selon la revendication 26 ou d'une composition selon la revendication 15, pour la fabrication d'un médicament destiné à traiter la malnutrition.

32. Utilisation d'un composé selon la revendication 26 ou d'une composition selon la revendication 15 pour la fabrication d'un médicament destiné à traiter l'anorexie.

33. Utilisation selon l'une quelconque des revendications 30 à 32, dans laquelle ledit médicament est destiné à l'administration orale.
